# EUROPEAN PATENT APPLICATION

(11) **EP 4 397 273 A2**
(43) Date of publication of application: **10.07.2024**
(21) Application number: 24151050.2
(22) Date of filing: 09.01.2024
(51) Int. Cl.: A61F 2/01

(54) **SURGICAL SYSTEM FOR A HEART VALVE**

(30) Priority: 09.01.2023 US 202363479167 P
(71) Applicant: Excision Medical, Inc., Malvern, PA 19355 (US)
(72) Inventor: WALTERS, Greg A., Malvern, 19355 (US); SMITH, Chad J., Phoenixville, 19460 (US); KANE, Alison Mikayla, Malvern, 19355 (US); CHERIAN, Shawn Sabu, Malvern, 19355 (US); DOTSEY, Michael A., Malvern, 19355 (US); KEISER, Jason, Malvern, 19355 (US); HARDER, Lucas, Malvern, 19355 (US); HAARSTAD, Lucas, Malvern, 19355 (US); HAARSTAD, Philip J., Chanhassen, 55317 (US); RADZWILL, Elizabeth, Malvern, 19355 (US)
(74) Representative: Haseltine Lake Kempner LLP

(57) **Abstract**

The present disclosure describes a surgical system for a heart valve. The surgical system is configured to serve as a temporary valve and/or filter to facilitate maintenance of proper blood flow while also capturing debris of an excised leaflet as needed.

## Description

### TECHNICAL FIELD

The present disclosure relates to a surgical system for maintaining proper blood flow during or after excision of portions of a heart valve, whether native or implanted.

### BACKGROUND

Transcatheter aortic valve replacement (TAVR) is an alternative option for the treatment of patients with severe calcific aortic stenosis. Indeed, TAVR may become the preferred therapy for all patients irrespective of surgical risk. However, transcatheter heart valves (THV) may fail in the future and repeat intervention may be required. So-called redo-transcatheter aortic valve implantation (TAVI) or TAVR may lead to risks of coronary obstruction due to the leaflet of the failed valve being pushed up by the new valve and leading to obstruction of blood flow to the coronary arteries. TAVR in failed surgical bioprostheses is common. However, TAVR in failed transcatheter bioprostheses (i.e. transcatheter heart valve-in-transcatheter heart valve) will also become increasingly common. In both situations there is a risk of coronary obstruction. The risk of coronary obstruction may be predicted with the use of cardiac computed tomography. If the predicted risk of coronary occlusion is high, then percutaneous valve-in-valve intervention may be prohibitive. In some cases, the cause of the coronary obstruction is related to the leaflets of the failed surgical or transcatheter heart valve that are pushed up and prevent flow of blood to the coronary arteries.

### SUMMARY

There is a need for systems, devices, and procedures for maintaining proper blood flow during excision of portions of a valve, whether implanted or a native heart valve. As such, an embodiment of the present disclosure is a surgical system. The surgical system includes an outer shaft having a lumen, and an inner shaft carried in the lumen such that one or both the outer shaft and the inner shaft are movable relative to each other. The surgical system includes an expandable valve assembly including a barrier within the expandable valve assembly and configured to selectively permit or inhibit fluid flow through the expandable valve assembly when in an expanded configuration. The expandable valve assembly being coupled to the outer shaft and the inner shaft, such that, movement of one or both the outer shaft and the inner shaft cause the expandable valve assembly to transition between a collapsed configuration and the expanded configuration.

Another embodiment of the present disclosure is a surgical system that includes an outer shaft having a lumen and an inner shaft carried in the lumen such that one or both the outer shaft and the inner shaft are movable relative to each other. The surgical system includes an expandable valve assembly having a forward end and a trailing end, the forward end being coupled to the inner shaft and the trailing end being coupled to the outer shaft. In this configuration, movement of either or both the inner shaft and the outer shaft cause the expandable valve assembly to transition between a collapsed configuration and an expanded configuration.

Another embodiment of the present disclosure is a surgical system. The surgical system includes an outer shaft having a lumen and an inner shaft carried in the lumen such that one or both the outer shaft and the inner shaft are movable relative to each other. The surgical system includes an expandable valve assembly coupled to one or both of the outer shaft and the inner shaft. Movement of one or both the outer shaft and the inner shaft is configured to 1) cause the expandable valve assembly to transition between a collapsed configuration and an expanded configuration, and 2) selectively control an outer cross-sectional dimension of the expandable valve assembly. This may result in selectively adjusting the radial forces applied outwardly by the expandable valve assembly.

Another embodiment of the present disclosure is a surgical system. The surgical system includes an expandable valve assembly having a forward end, a trailing end, an outer wall, a flexible skirt that at least partially surrounds the outer wall, and at least one void between the outer wall and the flexible skirt. The surgical system includes a barrier within the expandable valve assembly and configured to selectively transition between an open configuration, where the barrier is collapsed inwardly, and a closed configuration, where the barrier is expanded outwardly adjacent to the outer wall. The flexible skirt is configured to conform to the outer wall adjacent the at least one void to create passage external to the flexible skirt that permits an adjacent catheter to enter alongside an expandable valve assembly.

Another embodiment of the present disclosure is a surgical system. The surgical system includes an outer sheath having a lumen and an outer shaft carried in the lumen such that one or both the outer sheath and the outer shaft are movable relative to each other. The surgical system also includes an expandable valve assembly including forward end, a trailing end, and a barrier within the expandable valve assembly and configured to selectively permit or inhibit fluid flow through the expandable valve assembly when in an expanded configuration, the forward end of the expandable valve assembly being coupled to the outer shaft and the trailing end being uncoupled to the outer shaft, such that, movement of the outer sheath relative to the outer shaft causes the expandable valve assembly to transition between a collapsed configuration and an expanded configuration.

Another embodiment of the present disclosure is a method. The method includes advancing an expandable valve assembly in a collapsed configuration into a heart to a location proximate to a heart valve. Here, the expandable valve assembly is coupled to an outer shaft and an inner shaft. The method also includes actuating movement of one or both the outer shaft and the inner shaft to transition the expandable valve assembly from the collapsed configuration into an expanded configuration. The method also includes controlling blood flow through the expandable valve assembly via blood flow responsive opening and closing of a barrier contained within the expandable valve assembly when in the expanded configuration. The method includes collapsing the expandable valve assembly into collapsed configuration and retracting the expandable valve assembly in the collapsed configuration from the location proximate to the heart valve.

Another embodiment of the present disclosure is a method. The method includes advancing an expandable valve assembly in a collapsed configuration to a location in an ascending aorta that is proximal to a heart valve. The method includes actuating the expandable valve assembly to transition from the collapsed configuration into an expanded configuration. Here, the expandable valve assembly has an outer wall, a flexible skirt, and a void formed between the outer wall and the flexible skirt. The method also includes inserting a separate catheter into a passage formed between the ascending aorta and an outer surface of the flexible skirt, wherein the space is located where the flexible skirt conforms to the outer wall at the void of the expandable valve assembly to define the passage. The method includes performing a surgical procedure with the separate catheter. The method includes collapsing the expandable valve assembly into the collapsed configuration. The method includes retracting the expandable valve assembly in the collapsed configuration from the ascending aorta.

### BRIEF DESCRIPTION OF THE DRAWINGS

The foregoing summary, as well as the following detailed description of illustrative embodiments of the present application, will be better understood when read in conjunction with the appended drawings. For purposes of illustrating the present application, the drawings show exemplary embodiments of the present disclosure. It should be understood, however, that the present disclosure is not limited to the precise arrangements and instrumentalities shown in the drawings. In the drawings:
Figure 1 is a schematic sectional view of a heart;
Figure 2A is an exploded side view of a surgical system for maintaining proper blood flow during or after excision of portions of an aortic valve according to an embodiment of the present disclosure;
Figure 2B is a side perspective view of the configurable section of the surgical system illustrated in Figure 1 in an expanded state;
Figure 3 is a top perspective view of the configurable section of the surgical system shown in Figure 1 in an expanded state;
Figure 4 is a cross-sectional view of a barrier according to an embodiment of the present disclosure;
Figure 5 is a top perspective view of the configurable section of the surgical system illustrated in Figure 1 in an expanded state with a barrier according to an embodiment of the present disclosure;
Figure 6A is a cross sectional view of the barrier shown in Figure 5 in an aorta having a large inner diameter;
Figure 6B is a cross sectional view of the barrier shown in Figure 5 in an aorta having a small inner diameter;
Figure 7A is a cross sectional view of a barrier according to an embodiment of the present disclosure;
Figure 7B is a cross sectional view of a barrier according to an embodiment of the present disclosure;
Figure 8 is a perspective view of a surgical system according to another embodiment of the present disclosure;
Figure 9A is a perspective view of an expandable valve assembly of the surgical system shown in Figure 8;
Figure 9B is an end view of the surgical system shown in Figure 9A;
Figure 9C is a side view of the surgical system shown in Figure 9A;
Figure 9D is a schematic side sectional view of the surgical system shown in Figure 9A, showing an expandable valve assembly in collapsed position retracted in an outer sheath;
Figure 9E is a schematic side sectional view of the surgical system shown in Figure 9A, showing an expandable valve assembly in collapsed position positioned outside of the outer sheath;
Figure 9F is a schematic side sectional view of the surgical system shown in Figure 9A, showing an expandable valve assembly in expanded position;
Figure 9G is a side view of the surgical system shown in Figures 9A, illustrated positioned inside an inferior portion of an implanted valve below the valve leaflets;
Figure 10A is a detailed view of a handle of the surgical system shown in Figure 8;
Figure 10B is a partial sectional of the handle of the surgical system shown in Figure 10A;
Figure 10C is a perspective view an actuator of the surgical system shown in Figure 10A;
Figure 10D to a bottom perspective view of an actuator used in the handle of the surgical system shown in Figure 10A;
Figure 10E is a partial detailed sectional view of the handle of the surgical system shown in Figure 10B;
Figure 10F is a partial detailed sectional view of the handle of the surgical system shown in Figure 10B;
Figure 10G is sectional view of the handle of the surgical system shown in Figure 10B;
Figure 10H is a perspective view an actuator of the surgical system shown in Figure 10A;
Figure 11A is a schematic side sectional view a surgical system shown in Figure 9A, illustrated positioned in an inferior portion of a valve at a location inferior to the coronary ostia as shown in Figure 9G;
Figure 11B is a schematic side sectional view a surgical system according to another embodiment, illustrated positioned in a valve and partially in the left ventricular outflow tract (LVOT);
Figure 12 is a schematic end view of a surgical system, at a location inferior to the implanted or native valve, according to another embodiment of the present disclosure;
Figure 13 is a schematic side sectional view of a surgical system according to another embodiment of the present disclosure;
Figure 14 is a schematic side sectional view of a surgical system according to another embodiment of the present disclosure;
Figure 15 is a detailed schematic side sectional view of a surgical system shown in Figure 14;
Figure 16 is a schematic side sectional view of a surgical system according to another embodiment of the present disclosure;
Figure 17 is a schematic side sectional view of a surgical system according to another embodiment of the present disclosure;
Figure 18 is a schematic side sectional view of a surgical system according to another embodiment of the present disclosure, shown positioned in the LVOT;
Figure 19 is a schematic side sectional view of a surgical system according to another embodiment of the present disclosure;
Figure 20 is a schematic side sectional view of a surgical system according to another embodiment of the present disclosure;
Figure 21 is a schematic side sectional view of a surgical system according to another embodiment of the present disclosure;
Figure 22 is a schematic side sectional view of a surgical system according to another embodiment of the present disclosure;
Figure 23 is a schematic side sectional view of a surgical system according to another embodiment of the present disclosure;
Figure 24 is a schematic end view of a valve frame of a surgical system according to another embodiment of the present disclosure;
Figure 25 is a schematic end view of a surgical system according to another embodiment of the present disclosure, illustrating a barrier in a collapsed or open state and a separate catheter inserted adjacent to the surgical system;
Figure 26 is a schematic end view of a barrier of a surgical system according to another embodiment of the present disclosure;
Figure 27 is a schematic end view of the surgical system shown in Figure 25, illustrating the barrier in an expanded or closed state, and a separate catheter inserted adjacent to the surgical system;

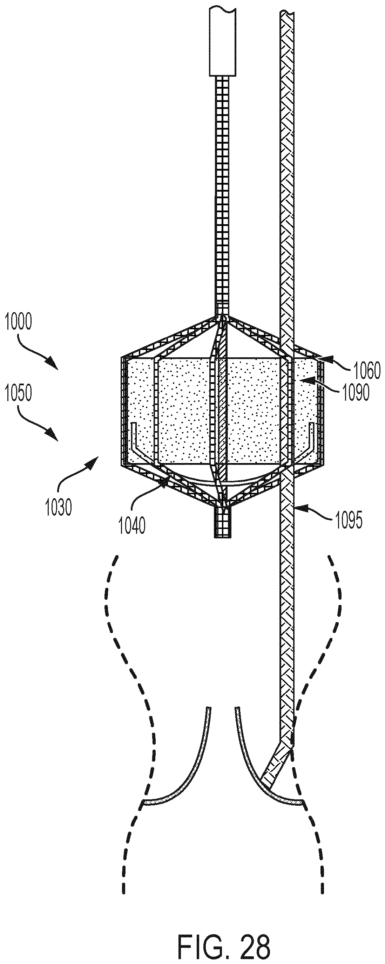

Figure 28 is a schematic side view of the surgical system shown in Figure 25, illustrating the barrier in an expanded or closed state, and a separate catheter inserted adjacent to the surgical system;
Figure 29A is a schematic side sectional view of a surgical system showing an expandable valve assembly with tethers, according to another embodiment of the present disclosure;
Figure 29B is a schematic side sectional view of a surgical system showing an expandable valve assembly with tethers, according to another embodiment of the present disclosure;
Figure 29C is a schematic side sectional view of a surgical system showing an expandable valve assembly with a tether, according to another embodiment of the present disclosure;
Figure 29D is a schematic side sectional view of a surgical system showing an expandable valve assembly with a tether, according to another embodiment of the present disclosure;
Figure 29E is a schematic side sectional view of a surgical system showing an expandable valve assembly with a tether, according to another embodiment of the present disclosure; and
Figure 29F is a schematic side sectional view of a surgical system showing an expandable valve assembly with tethers, according to another embodiment of the present disclosure;

### DETAILED DESCRIPTION OF ILLUSTRATIVE EMBODIMENTS

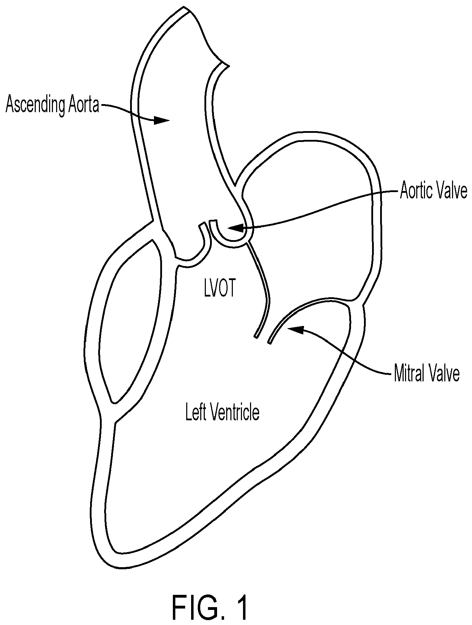

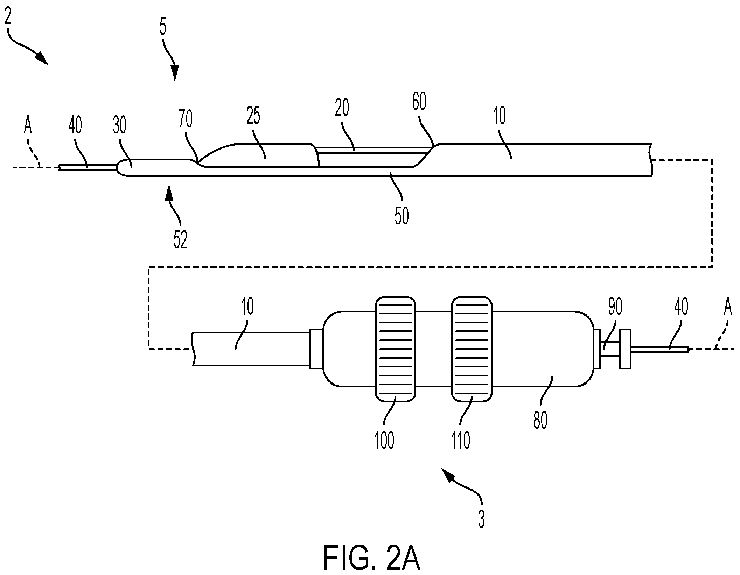

Figure 1 illustrates the anatomy of a heart, which includes the ascending aorta, the aortic valve, and LVOT adjacent to the left ventricle (LV) and the mitral valve. The left ventricular outflow tract is a portion of the left ventricle through which blood passes in order to exit through the aortic valve into the aorta. As shown in Figures 2A-2B, in the illustrated embodiments described herein, a surgical system 2 is intended to provide temporary hemodynamic protection during or after index procedures involving the cutting, excising, or manipulation of aortic valve leaflets. In cases where the surgical system is used during index procedures, the index catheter (TAVR, leaflet excision system, etc.) is present alongside or adjacent to an expandable shield or barrier. Alternatively, the surgical system 2 may be used in cases other than aortic valve excision procedures, including, for example, in cases where aortic valve hemodynamic support is needed. The surgical system 2 may include an expandable shield or barrier that may function as a temporary one-way valve to facilitate proper blood flow. The barrier is configured to enable adequate forward flow with the left ventricle (LV) ejection of blood flow, by opening in response to blood flow. The barrier and its one-way valve type configuration are designed to limit diastolic flow in the direction of the LV. Examples of such surgical systems are disclosed in U.S. Application Serial No. 18/379,390, the entire contents of which are incorporated herein.

The surgical system 2 may include a filter (not depicted) configured to capture debris as needed. The filter may include one or more movable panels (or other structures) that are responsive to fluid flow or fluid impinging the panels in order to manage blood flow in the aorta. The filter may also be used to extract and capture emboli, such as water vapor, char, smoke, oxygen, nitrogen, carbon dioxide, solids, tissue fragments, etc. In one example, the filter may be positioned to appose the aortic wall in a manner that captures particles from the forward flow ejection of the left ventricle (LV).

The surgical system may be used in combination with additional devices that are configured to a guide, capture, cut, and remove a portion of the leaflet of the valve, as described in U.S. Provisional Patent Application Serial No. 63/324,413, filed March 28, 2022, U.S. Provisional Patent Application Serial No. 63/022,119, filed May 8, 2020, U.S. Provisional Patent Application Serial No. 62/944,109, filed December 5, 2019, U.S. Provisional Patent Application Serial No. 63/176,507, filed April 19, 2021, and U.S. Patent Application Serial No. 17/782,238, filed June 3, 2022, the entire contents of which are incorporated by reference to into the present disclosure.

Continuing with Figures 1-2B, the system 2 as described herein are configured to provide access to an aorta. The surgical system 2 may therefore include one or more distinct elements designed to guide the system toward and into the aorta and position the barrier in place. More specifically, the surgical system 2 may include one or both of the following elements, either combined in a single assembly or comprising separate modular components: (a) a catheter or shaft 10 for targeting the system 2 toward the desire tissue site; (b) a plurality of struts 20 that are responsive to fluid flow; and (c) a barrier or expandable shield 25. The surgical system may include a handle and may include one or more actuators that are configured to control operation of and relative movement of elements ((a) through (c) above) of the system 2 in use.

The surgical system 2 is generally sized and configured for insertion into a sheath positioned in the aorta. The system 2 may include additional devices, such as guide wires, introducers, etc., to facilitate introduction of the surgical system into the aortic arch. In terms of size, the distal end and shafts of the system 2 may be sized to fit within a sheath. For example, the surgical system shaft may have an outer diameter, measured perpendicular to a central axis 1 thereof, up to about 14F. In one embodiment, the sheath may be a TAVR sheath. In another embodiment, the sheath may be an additional access sheath having a proximal end, a distal end spaced from the proximal and a lumen that extends from the proximal end to the distal end. The inner diameter of the sheath is sized to fit around a guidewire that may be at least 0.035 inches. Furthermore, the effective length of the surgical system 2, such as the portion that extends from the entry site of a patient to the target location in the aorta may vary. In some examples, the effective length may range between about 40 cm up to about 100 cm, and any intervals therebetween. In other examples, the effective length may be larger than 100 cm. Accordingly, the surgical system size and configuration could vary as needed.

The surgical system 2, and specifically the one or more elements described above ((a) through (c) and further described below) include an elongate conformable shaft 10 that engages or is coupled to the handle and is designed to extend into the aortic arch, either alone, or coupled to a guidewire, which is typically placed in the ascending arch of the aorta to provide access to the aortic valve. The surgical system is also designed to pass through a procedural sheath. The elongate shaft may be in the form of a catheter, which includes an internal channel through which other devices and elements or may pass. Its form as a catheter is useful, as needed, when coupled with other surgical devices for access to and engagement with an implanted valve in the aorta.

As illustrated in Figures 2A-2B, the elongated catheter 10 includes a proximal end 3 and a distal end 5 that are spaced apart from each other along a central axis A. The elongated catheter 10 is therefore elongated along the central axis A. Generally, a direction from the proximal end 3 toward the distal end 5 is referred to herein as the distal direction. A direction from the distal end 5 toward the proximal end 3 is referred to herein as the proximal direction (opposite the distal direction). The elongated catheter 10 includes a distal tapered tip 30 on the distal end 5. The distal tapered tip 30 on the distal end 5 is coupled to the plurality of struts 20 at a terminal end of the plurality of struts 70 (shown in Figure 2A). The distal tapered tip 30 is further coupled to the barrier 25.

The elongated catheter 10 includes a configurable section (not numbered) spaced from the distal end 5. The configurable section is configured to selectively conform to a curvature of an aortic arch when the surgical system 2 is in a closed configuration in the aortic arch. The configurable section is sized such that the entirety of the configurable section may be positioned in various anatomical positions including in or downstream of the left ventricular outflow track and upstream of the cerebral vasculature extending from the aortic arch.

The elongated catheter 10 includes a conformable main shaft 50, that may be selectively fixed in a curved configuration, disposed along the length of the elongated catheter 10 from the proximal end 3 to the distal end 5. The main shaft 50 includes a terminal end 52 disposed at the distal end 5. The terminal end 52 is coupled to the distal tapered tip 30 of the elongated catheter 10. The main shaft 50 extends along the central axis A parallel to the struts 20 and barrier 25. The main shaft 50 is sized and shaped to fit a guidewire lumen that extends through the main shaft 50 from the proximal end 3 to the distal end 5. The guidewire lumen is configured to receive a guidewire 40 therethrough. In the illustrated embodiment, the guidewire 40 may be at least 0.035 inches. In alternative embodiments, the guidewire may be less than 0.035 inches. The elongated catheter 10 is ideally situated between the moveable struts 20 on the outside curvature of the aorta, while the main shaft 50 is shaped to be along the inside curvature of the aorta.

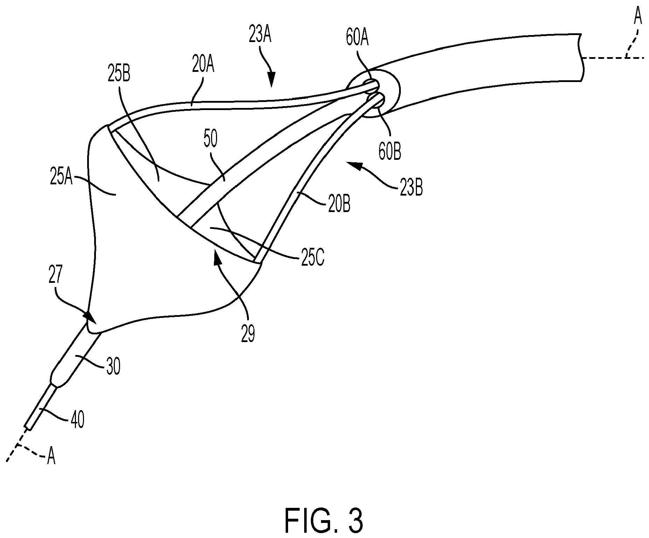

The elongated catheter 10 further includes one or more lumen 60 configured to extend longitudinally along the central axis A and parallel to the main shaft 50 and to each other. The lumen 60 partially contain the struts 20 and allow the struts to slide inside the lumen 60 from an open configuration to a closed configuration. In the illustrated embodiment, the elongated catheter includes a first lumen 60A and a second lumen 60B (shown in Figure 3). The first lumen 60A is disposed on the catheter 10 and the second lumen 60B is disposed on the catheter 10 and spaced from and parallel to the first lumen 60A.

The shafts described herein, when in the form of catheters, will generally include a shaft, an inner channel, one or more radiopaque markers, and a distal tip. One of or more catheters as described herein may have a secondary curve, a primary curve, a configurable curve, or no pre-set curves. The primary, secondary, and configurable curves are not illustrated in the drawings. The distal tip defines the distal most end of each elongate shaft. The shaft may, for example, include an inner channel that is also sized to receive other surgical devices therethrough.

For example, the surgical system 2 may receive the guidewire 40 such that an over-the-wire technique may be used. That is, the guidewire 40 may be placed into the aorta, and the surgical system 2 may be inserted over the guidewire 40 into position via the distal tapered tip 30 and main shaft 50. In an alternative embodiment, the surgical system 2, or one or both of its shafts, may include one or more skive ports that may be used to receive the guidewire 40 therethrough. Such skive ports may be disposed toward or along an outer surface of the shaft. In yet another embodiment, the guidewire may not extend through the main shaft into the aorta. The surgical system 40, however, may still slide over or along the guidewire, but without the benefit of having the guidewire 40 cross through the main shaft.

In cross-section, a catheter may include an inner liner, a middle reinforcing layer (e.g. a braid), and an outer layer or outer jacket. In addition, the catheter may be a biaxial design that includes an additional outer layer to minimize interaction with the introducer and/or sheath and allow smoother movement of the surgical system.

The longitudinal shape of the catheter may vary as needed. For instance, the catheter may have a shape according to the Amplatz Guide that includes, but is not limited to AL-1, AL-2, AL-3, AL-4, etc. Other common shapes are possible as well. In one example, the catheter may have an outer cross-sectional dimension sized for insertion into the aorta. For instance, the catheter may be either 12 French or 14 French. However, larger or smaller sized catheters may be used in certain instances. The catheter tip, distal tip, and/or configurable section may be deflectable or bendable as needed to fix the distal portion of the catheter into position.

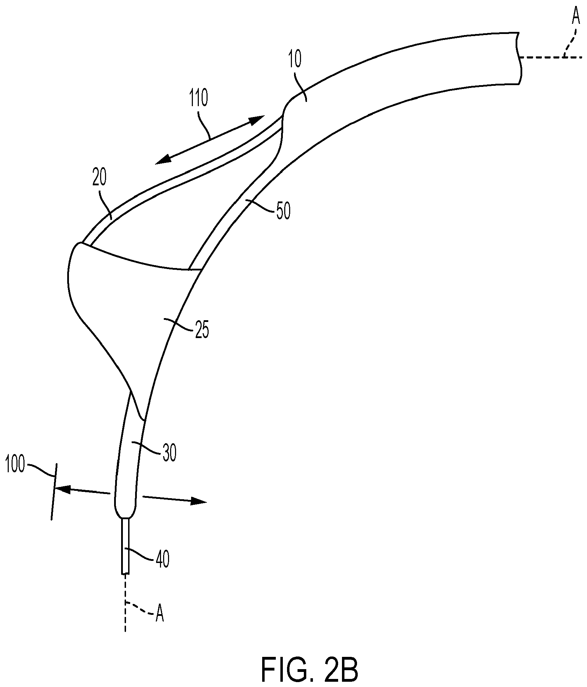

Continuing with Figures 2A and 2B, the catheter 10 has at least one port 90 that extends to the inner channel. The at least one port 90 may be two or more ports as needed. The port or ports 90 are spaced a distance from the proximal end 3 that is less than a distance between the at least one port 90 and the distal end 5. In other words, they are positioned toward the proximal end 3 of the catheter 10. These ports 90 are intended to a) allow for flushing or priming the system prior to introduction to the patient and/or b) to provide for hemodynamic monitoring of the blood pressure in the ascending aorta. For instance, when the leaflets get cut, the destruction of the aortic valve may lead to decompensation of cardiac output, which is monitored by a local lumen. In one example, the system 2, may, in turn, include a luer fitting on the handle for monitoring and flushing the system.

The surgical system 2 includes a handle 80 disposed at the proximal end of the elongated catheter 10. The handle 80 may include one or more actuators disposed on the handle to control transition of the actuatable panel between and among the retracted or closed configuration and the expanded or open configuration. More specifically, in the illustrated embodiment, the surgical system 2 includes a first actuator 110 and a second actuator 100. The first actuator 110 is coupled to the struts 20. The first actuator 110 is configured to control operation of the struts 20 to permit or inhibit fluid flow through the barrier 25. In the illustrated embodiment, the actuator 110 is a knob such that when the knob is rotated, the struts 20 are configured to slide along the lumen 60 and actuate the barrier 25 to transition into an closed configuration.

The second actuator 100 is configured to cause the configuration of the configurable section to selectively change. In the illustrated embodiment, the second actuator 100 may include one or more push-pull rods or wires coupled to either the configurable section of the elongated configurable catheter 10 or coupled to the distal tip 30. In one example, activation of one or more push-pull rods or wires causes the configurable section to curve into and out of a curved configuration. For example, one or more push-pull rods or wires may be located in a lumen inside the main shaft 50, anchored in the distal tip 30, and actuated by the handle 80. The configurable section, and a distance proximal to the configurable section may curve in at least one plane when tension is applied to the one or more push-pull rods or wires.

Referring to Figure 3, the surgical system 2 includes a plurality of separate flexible arms or struts 20 coupled to the elongated catheter 10 and the barrier 25. In the illustrated embodiment, the surgical system 2 includes a first strut 20A and a second strut 20B. The first strut 20A is slidable along the first lumen 60A of the elongated catheter 10. The first strut 20A has a terminal end (not depicted) disposed near the distal tapered tip 30, and an intermediate section 23A that extends from the terminal end and into the first lumen 60A. The surgical system 2 further includes a second strut 20B that is slidable in the second lumen 60B. The second strut 20B has a terminal end (not depicted) disposed near the distal tapered tip 30, and an intermediate section 23B that extends from the terminal end and into the second lumen 60B. The first strut 20A and the second strut 20B are movable relative to each other and the main shaft 50. The coupling location of the first strut 20A and the second strut 20B to the main shaft 50 controls orientation of the first strut 20A and the second strut 20B. The terminal ends are coupled to the distal tapered tip 30.

The intermediate sections 23A, 23B of the struts 20A, 20B are configured to exit the respective first and second lumens 60A, 60B at a preset angle (circumferential spacing) relative to each other, at a preset shape, and at a range of distances controllable via an actuator, into a configuration that is spaced outwardly away from the main shaft 50. In one example, the preset angle of the first strut 20A, and the second strut 20B is between 90 degrees and 180 degrees. In another example, the preset angle of the first strut 20A and the second strut 20B is at least 90 degrees. This configuration enables adjusting of the struts 20A, 20B as they exit the lumens 60A, 60B to conform with various inner diameters of aortas.

Continuing with Figure 3, the surgical system 2 further includes a barrier 25 carried by the shaft. In the illustrated embodiment, the barrier 25 has a leading end 27 defining a tapered tip and coupled to the distal tapered tip 30 and a trailing end 29 that is proximal relative to the leading end 27 and coupled, either movably or fixed, to each of the first strut 20A and the second strut 20B. The barrier 25 is movable between an open configuration, where the barrier 25 is collapsed toward the central axis, and a closed configuration, where the trailing end 29 expands outwardly from the central axis A in response to a second direction of fluid flow.

The barrier 25 may be divided into one or more leaflets. In the illustrated embodiment, the barrier 25 is divided into three barrier leaflets 25A, 25B, 25C. In alternative embodiments, the number of barrier leaflets 25 may vary. The barrier 25 is coupled at one end to the main shaft 50 and the distal tapered tip 30 and is moveably coupled to the intermediate sections of the first strut and the second strut 23A, 23B respectively, such that, advancement of the first strut 20A and the second strut 20B in the distal direction causes the intermediate sections of the first strut and the second strut 23A, 23B respectively to expand outwardly in order to maintain the barrier 25 in the closed configuration.

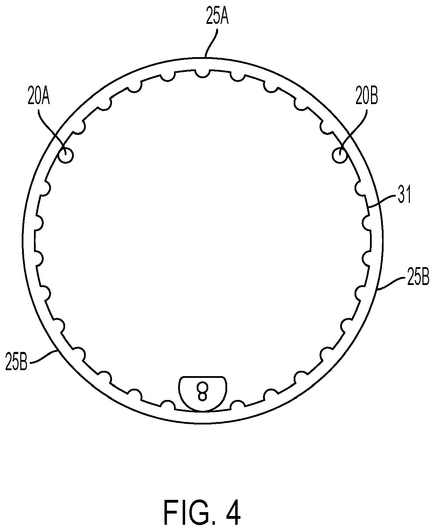

Referring to Figure 4, in one embodiment, the barrier 25 may include longitudinal ribs 31 along an internal surface of the barrier 25. The ribs 31 may be attached or adhered to the barrier 25 in various ways that are known to one skilled in the art. In one example, the ribs 31 may be molded to the barrier 25. In another example, the ribs 31 may be adhered or machine-attached to the barrier 25. This configuration allows the barrier 25 to have increased longitudinal stiffness and may also prevent prolapsing or inverting of the barrier 25.

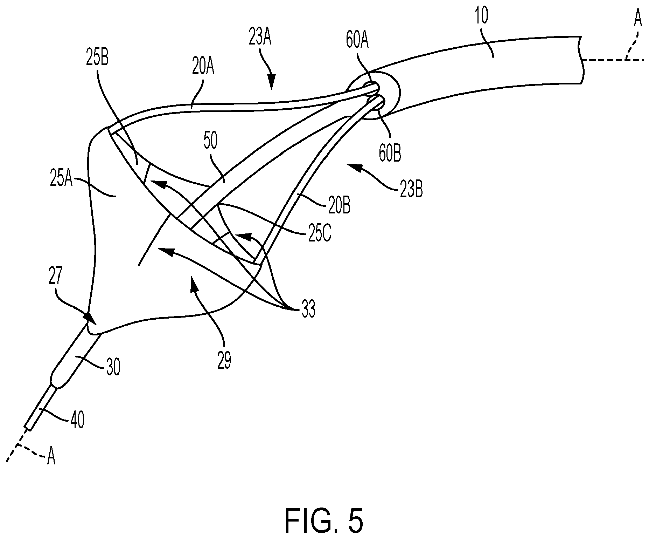

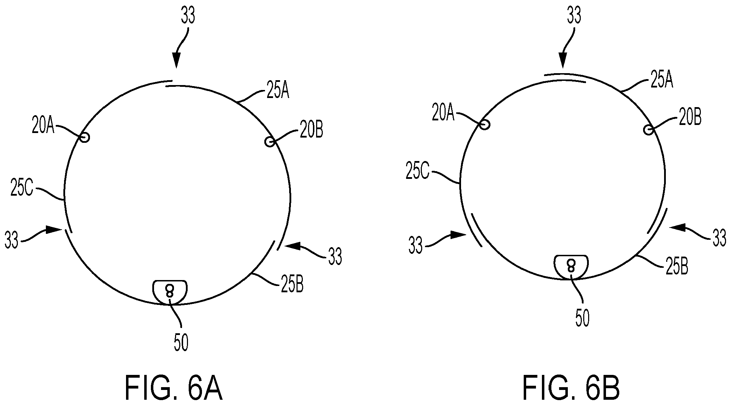

Referring to Figures 5-6B, in another embodiment, the barrier 25 may include one or more longitudinal slits 33 disposed at the trailing end. In the illustrated embodiment, the barrier 25 includes one slit 33 along each of the leaflets 25A, 25B, 25C. This configuration allows the slitted portion of the leaflets 25A, 25B, 25C to overlap and maintain a seal when deployed in aortas of varying internal diameters. For aortas with larger inner diameters, the leaflets 25A, 25B, 25C containing longitudinal slits 33 may have minimal overlap, as shown in Figure 6A. For aortas with smaller inner diameters, the leaflets 25A, 25B, 25C containing longitudinal slits 33 may have larger overlap, as shown in Figure 6B.

In another embodiment, the barrier 25 may include an opening in one or both of the leaflets 25A, 25B, 25C. The opening is configured to accommodate passage of an index catheter body (for example, TAVR, leaflet excision system, etc.) through the barrier 25, as opposed to alongside or adjacent to the barrier 25. The opening may include a radiopaque feature to aid in angiographic guidance of the index catheter through the opening.

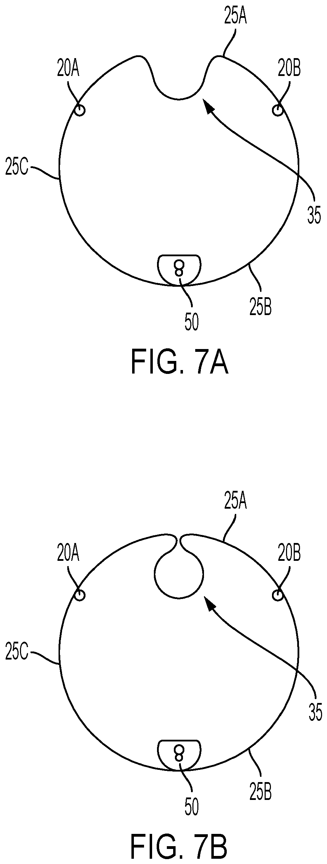

Referring to Figures 7A-7B, in another embodiment, the barrier 25 may include one or more channels 35 of various shapes disposed along one or more leaflets. In the illustrated embodiment, the barrier 25 includes one channel 35 along a single leaflet 25A. The one or more channels may include an opening having a radiopaque feature to aid in angiographic guidance of the index catheter through the opening. The one or more channels 35 may be a preformed shape and may include additional leaflet material spanning between two adjacent struts, or a strut and the main shaft 50, in order to accommodate an adjacent catheter body. The additional leaflet material may or may not coapt with the other leaflets 25A, 25B, 25C. This configuration allows the barrier 25 to seal around an adjacent catheter body. Furthermore, in one example, the portion of the barrier 25 between the first strut 20A and the second strut 20B may be larger than the portions of the barrier between the first strut 20A and the main shaft 50 and between the second strut 20B and the main shaft 50. In this example, the configuration allows the portion of the barrier between the first strut 20A and the second strut 20B to tuck in closer to the edges of the expandable catheter 10.

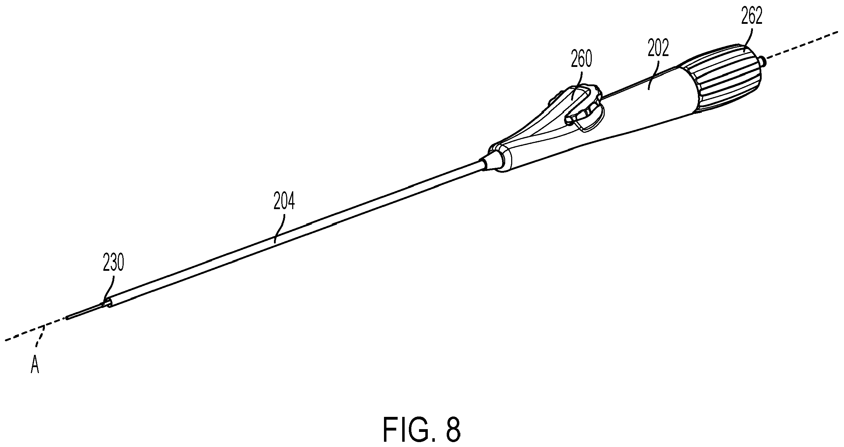

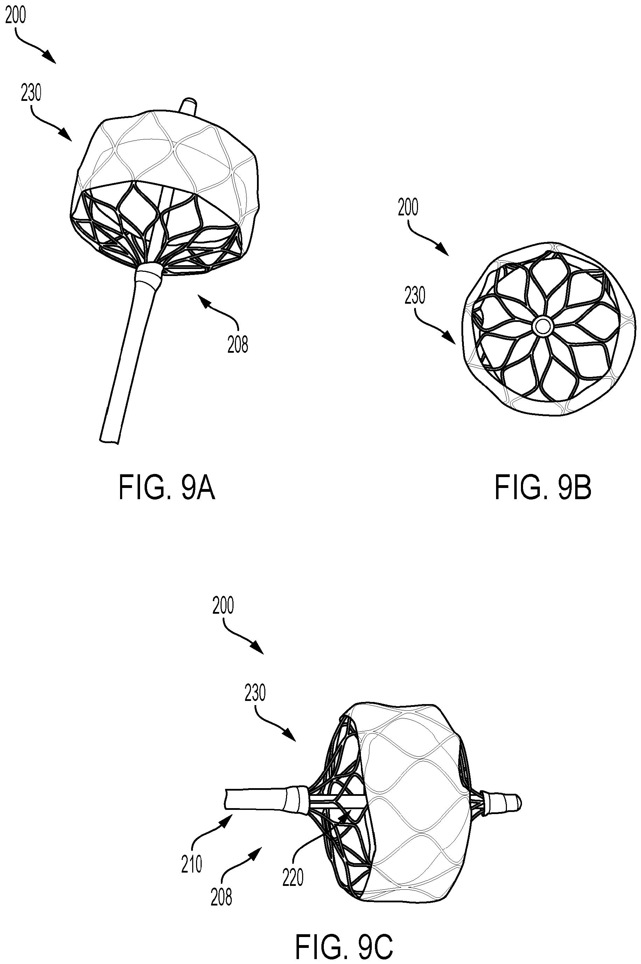

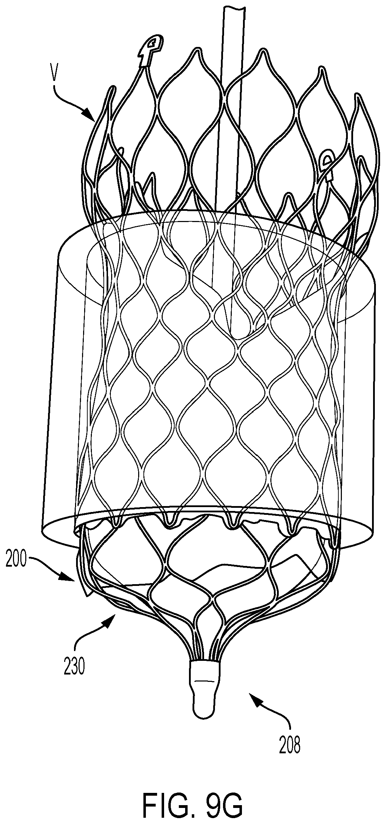

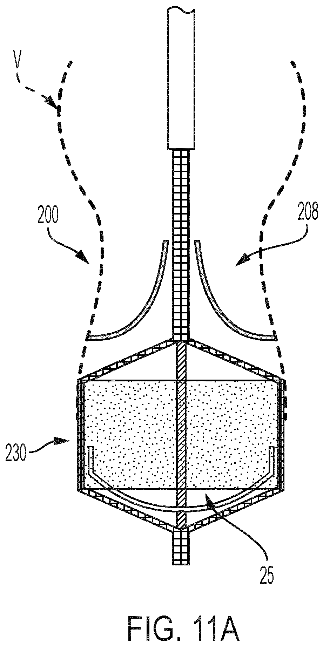

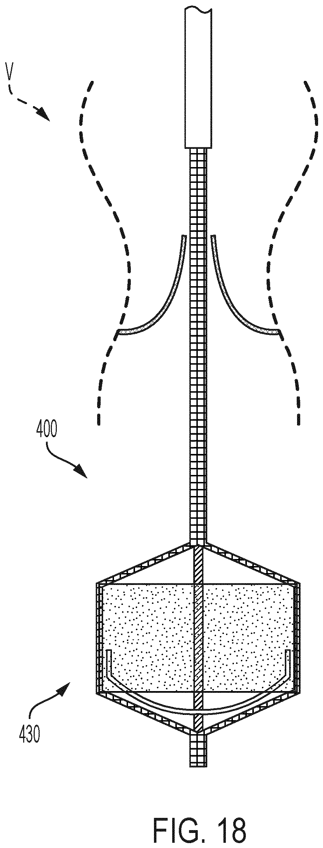

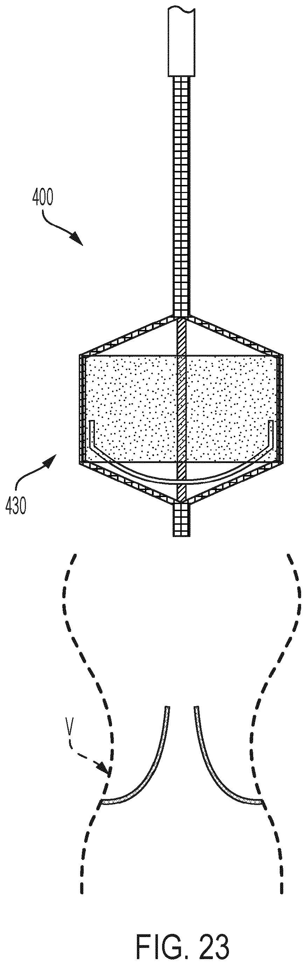

Figures 8 through 11A illustrate another embodiment of surgical system 200 according to the present disclosure. The surgical system 200 shown in Figures 8-11A may include features that are similar to the surgical system 10 described above and shown in Figures 1-7B. Similar reference numbers are used to identify features and elements that are common to surgical system 10 and surgical system 200. The surgical system shown in Figures 8-11A may be used to control blood flow from a location that is generally distal to (inferior to) the coronary ostia, or positioned "sub-coronary." In some cases, the surgical system 200 is positioned at least partially within an implanted valve, as shown in Figure 9G and 11A. In certain embodiments, the surgical system may be configured to function distal to the implanted valve in the LVOT, as shown in Figure 18. In certain embodiments, the surgical system may be configured to function proximal to the coronary ostia in the aorta, as shown in Figure 23. And in other cases, the surgical system may be configured to function distal to, inside, and proximal to native heart valves.

Referring to Figure 8, the surgical system 200 includes a handle 202, one or more actuators 260, 262, and an outer sheath 204 that carries the expandable valve assembly 230. The surgical system has a proximal end, defined by the handle 202, and a distal end that is opposite the proximal end along a longitudinal axis A.

Referring to Figures 8-11A, the surgical system 200 includes an outer sheath 204, an inner sub-assembly 208, and an expandable valve assembly 230. The surgical system 200 is configured to advance its distal end toward the target site, such as below the coronary ostia in an implanted valve and cause the expandable valve assembly 230 to expand outwardly for use, such as in controlling blood flow temporarily while additional procedures, such as excision of the valve leaflets using other medical device occurs.

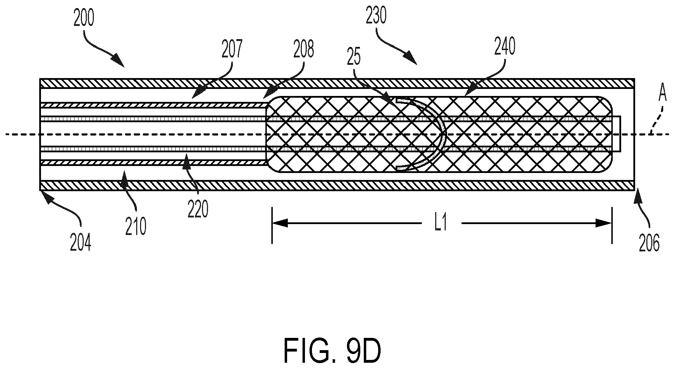

Continuing with Figures 8-11A, the outer sheath 204 has a distal end 206 and a channel 207 that extends therethrough. The outer sheath 204 may be configured as a catheter or outer catheter and may include components of a catheter as described elsewhere in the present disclosure. For example, the outer sheath may be steerable or may be guided to a particular location in the aorta or heart. Furthermore, the proximal end of the outer sheath may be coupled to a handle, which defines the proximal end of the surgical system 200 and is not shown in the figures. Both inner subassembly 208 and the expandable valve assembly 230 are moveable from within the channel 207 to a location outside of the channel 207 (as shown in the progression of Figures 9D-9F. For example, the inner subassembly 208 and the expandable valve assembly 230 are moveable from within the channel 207 to a location outside of the channel 207 by retracting the outer sheath. In another example, inner subassembly 208 and the expandable valve assembly 230 are moveable from within the channel 207 by advancing the inner subassembly 208 and the expandable valve assembly 230 relative to the outer sheath.

Continuing with Figures 8-11A, the inner sub-assembly 208 may include an outer shaft 210 having a lumen and an inner shaft 220 carried in the lumen. As shown, the outer shaft has a proximal end and a distal end with lumen extending from the distal end toward the proximal end. The inner shaft has a proximal end and a distal end. In some instances, the inner shaft may have a lumen configured to slide along a guide wire or another device for access to the target site. The inner shaft lumen may also be configured to 1) allow for radiopaque contrast injection or pressure monitoring, 2) aid in the delivery of a non-ionic solution, such as dextrose, to displace blood during electrification and concentrate electrical energy on the target leaflet when using an electrosurgical system for lacerating or excising a valve leaflet, or 3) deliver a cold flush of liquid to reduce the force required to collapse a valve assembly composed of nitinol. The inner shaft 220 may include a pigtail catheter portion on its distal end, with or without side holes. Each of the outer and inner shafts may extend longitudinally along an axis A. One or both of the outer shaft 210 and the inner shaft 220 are movable relative to each other to selectively cause expansion and collapse of the expandable valve assembly 230 as further explained below. The distal end of the outer sheath may be flared, expandable or comprised of a pliable material to ease recapture of the valve assembly.

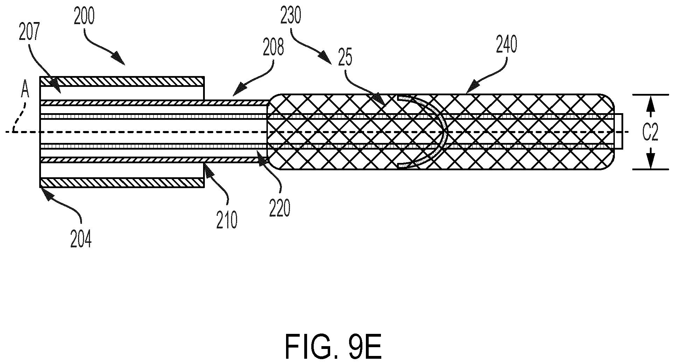

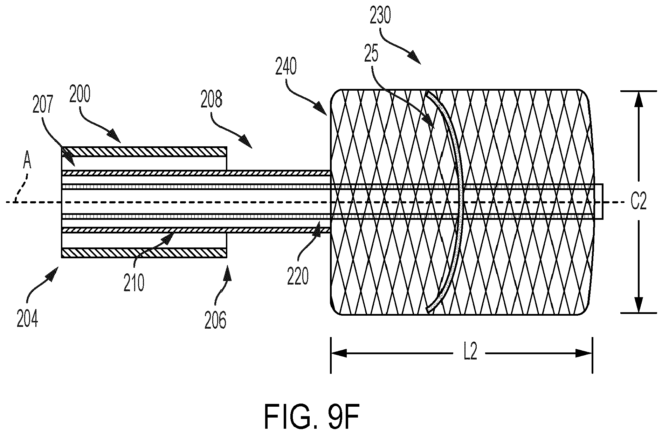

Referring to Figures 8-9F, the expandable valve assembly 230 generally includes a forward end, a trailing end spaced apart from the forward end along the central axis A, and an outer wall. The forward end of the expandable valve assembly is coupled to the distal end of the inner shaft and the trailing end of the expandable valve assembly that is coupled to the distal end of the outer shaft. Coupled in this manner, movement of either or both the inner shaft and the outer shaft relative to each other causes the expandable valve assembly transition between the collapsed configuration, as shown in Figures 9D and 9E, into the expanded configuration, as shown in Figure 9F. For example, in one embodiment, advancement of the outer shaft relative to the inner shaft in a distal direction causes the expandable valve assembly to transition from the collapsed configuration to the expanded configuration. In another example, retraction of the inner shaft relative to the outer shaft causes the expandable valve assembly to transition from the collapsed configuration to the expanded configuration. In yet another example, advancement of the inner shaft relative to the outer shaft in the distal direction causes the expandable valve assembly to transition from the expanded configuration into the collapsed configuration. In addition, retraction of the outer shaft relative to the inner shaft causes the expandable valve assembly to transition from the expanded configuration to the collapsed configuration. Thus, movement of one or the other of the outer and inner shafts, in either distal direction or proximal direction, may cause expansion and collapse of the valve assembly in use. In addition, the ability to expand and collapse the expandable valve assembly enables use in a range of vessel inner diameters and allows for control of the outward radial force to ensure positional stability without damaging the vessel or surrounding tissue.

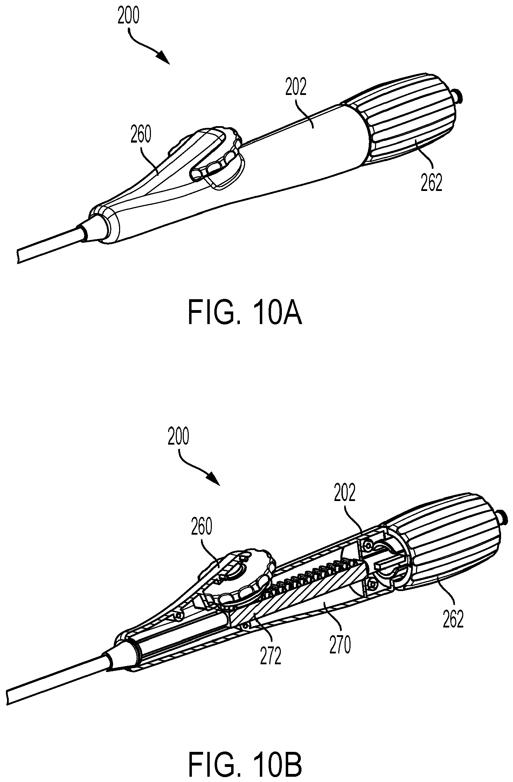

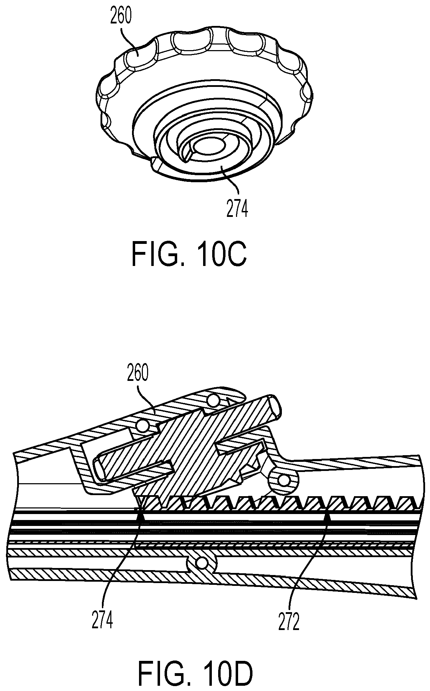

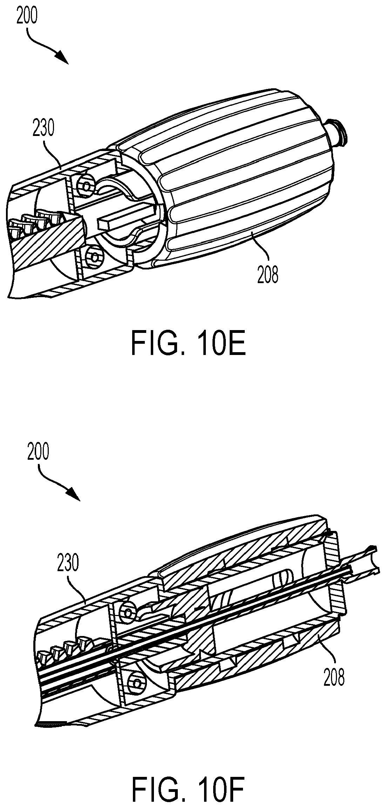

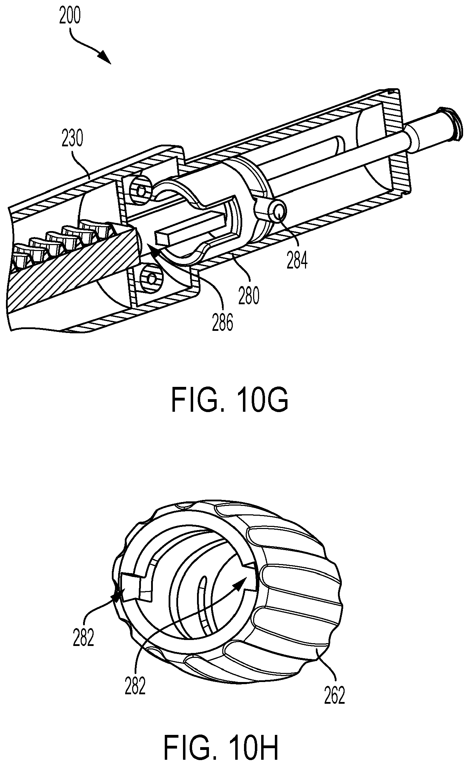

Figures 10A-10H illustrate an exemplary embodiment for controlling operation of the expandable valve assembly 230. As shown, the surgical system 200 may include sheath actuator 260, which controls movement of the outer sheath 204. The surgical system may include an inner valve subassembly actuator 262 that controls expansion and contraction of the expandable valve assembly 230.

The surgical system includes a control module 270 that engages the actuators 260 and 262. The control module 270 includes an outer sheath rack 272 that includes teeth and that is moveably coupled to a tapered spiral ridge scroll 274 on the underside of the actuator 260. The actuator 260 is configured as a wheel. The rack is fixed to the proximal end of the outer sheath. Rotation of the actuator 260 causes the rack to translate, which, in turn, causes the outer sheath 204 to translate as needed. The tapered scroll on the outer sheath control wheel engages with a rack that is bonded to the outer sheath. As the wheel is turned, the scroll advances and/or retracts the outer sheath 20 in a very controlled manner, requiring minimal force input by the user. While a rack and groove system is shown, other control mechanisms may be used to control translation of the outer sheath. For example, the actuator may be a slide that allows for control of the position of the outer sheath.

The control module 270 also includes a movable shuttle 280 coupled to the inside of the actuator 262. The actuator 262 is configured as a rotatable knob that rotates about axis A (not shown). The valve assembly 230 is opened and closed using the actuator 262 on the proximal end. The valve 230 is deployed and/or retracted by advancing and/or retracting the inner shaft. The inner shaft is bonded to a movable shuttle 280 which includes two lateral pins 284. The outer shaft is bonded to a fixed hub 286. The actuator 262 has two spiral grooves 282 cut into its inner surface. When the actuator 262 is turned, the spiral grooves 282 move the shuttle 280 via engagement with two opposing lateral pins 284, thereby causing the inner shaft to move/translate as needed. Accordingly, in this embodiment shown, rotation of the actuator 262 about the axis A cause the inner shaft to move along the axis A to control operation of the valve assembly 230. While the features are shown with a particular configuration, control of the inner and outer shaft may be via any number of different mechanisms as needed. In the illustrated embodiment, the diameter of the expandable valve assembly 230 and/or the radial outward force exerted by the expandable valve assembly may be selectively actuated using the handle 202.

In an embodiment of the present disclosure, the expandable valve assembly coupled to one or both of the outer shaft and the inner shaft, such that, movement of one or both the outer shaft and the inner shaft is configured to 1) cause the expandable valve assembly to transition between a collapsed configuration and an expanded configuration, and 2) selectively control an outer cross-sectional dimension of the expandable valve assembly, thereby selectively adjusting the radial forces applied outwardly by the expandable valve assembly. In this case, the user may control the precise outer dimensions, either during expansion or contraction, as needed.

The ability to selectively control an outer dimension during expansion/contraction has several benefits and differences over structure that rely solely on shape memory, such as stents and the like. For example, stents expand via shape memory of the stent and/or balloon expansion. The outward force of a stent is essentially preprogrammed based on the stent geometry (struts, apices, wall thickness, etc.) and the diameter of the stent relative to the vessel diameter. In present disclosure, the outer diameter of the valve assembly and the outward force it exerts is selectively controlled by the outer shaft/inner shaft movement. This attribute has multiple benefits. For example, the valve may be configured to work across a wider variety of anatomy, creating few SKU's for the surgeon and hospital system to manage. In addition, it increases the outward force to improve positional stability. This features also reduces outward force in delicate anatomy (i.e. a heavily calcified ascending aorta at risk of liberating emboli or in the LVOT where there is risk of inducing an arrythmia).

As shown in Figures 9D-9F, during expansion, the expandable valve assembly increases its cross-sectional dimension while its overall length is shortened. More specifically, the expandable valve assembly in the collapsed configuration has a first outer cross-sectional dimension C1 and expandable valve assembly in the expanded configuration and has a second outer cross-sectional dimension C2 that is greater than the first outer cross-sectional dimension. In the collapsed configuration, a length L1 of the expandable valve assembly is greater than a length L2 of the expandable valve assembly when in the expanded configuration.

In another embodiment of the present disclosure, the expandable valve assembly may be coupled at its forward end to the distal end of the outer shaft and is uncoupled at the trailing end, to provide a self-expanding configuration. More specifically, in such an embodiment, the inner sub-assembly may only include the outer shaft and the expandable valve assembly and no inner shaft is present.

In the embodiment shown, the expandable valve assembly 230 includes an expandable fame 240. The forward end defines a forward section that extends from the outer wall toward a forward tip that is coupled to the inner shaft, and the trailing end defines a trailing section that extends from the outer wall toward a trailing tip that is coupled to the outer shaft. As shown, the outer wall, forward section, and trailing section are formed by multiple frame members that define cellular voids. An optional skirt extends (at least partially) around the outer wall but does not overlay the forward section or the trailing section, thereby permitting blood to flow through the valve assembly. Alternatively, the skirt may be coupled to the internal surface of the valve frame, or both the external and internal surfaces. In addition, the skirt may overlay or extend over portions of the forward section and the trailing section to reduce paravalvular leakage.

The expandable frame 240 may be formed from a shape memory material. In one example, the frame 240 may be formed by laser cutting a tube or sheet of a shape memory material, such as nitinol and heating the frame in an expanded state such that its natural configuration is expanded. This may facilitate transition between the collapsed configuration and the expanded configuration during use as described below. In addition, the valve assembly may include a polymeric coating. Such a coating may help minimize electrical interference between the valve assembly with any RF cutting devices used to excise leaflets. In one embodiment, the expandable frame 240 may be positioned in a manner to move native or bioprosthetic valve leaflets into an orientation that enables or eases the laceration or excision of a valve leaflet by an electrosurgical system.

The expandable valve assembly 230 may include a barrier 25 (Figure 11A) within the expandable valve assembly that is configured to selectively permit or inhibit fluid (e.g. blood) flow through the expandable valve assembly when in the expanded configuration. The barrier 25 is movable between an open configuration, where the barrier 25 is collapsed toward the central axis A, and a closed configuration, where the trailing end 29 expands outwardly from the central axis A in response to a second direction of fluid flow. More specifically, in the open configuration, during systole flow, blood may pass through the expandable valve assembly, and in the closed configuration, during diastole, blood flow is substantially inhibited from passing through the expandable valve assembly.

In all embodiments of the present disclosure, the barrier 25 is comprised of a flexible membrane that is generally impermeable to blood. An exemplary barrier is made of polyurethane. The barrier 25 may be molded, heat formed, cut, or sewn into different geometries, such as a cone, dome, parachute, or plurality of flaps attaching at one or more points of the valve frame. In addition, the barrier may be attached to the central axis of the valve frame interior (collapsing inwards toward the central axis to open during systole and expanding outwards toward the frame to close during diastole). In other embodiments, the barrier may be attached along the outer circumference of the valve frame interior (expanding outwards toward the frame to open during systole and collapsing inwards toward the central axis to close during diastole). Prolapse of the barrier may be mitigated by allowing the barrier to coapt onto the frame at a 90 degree angle or below from the central axis. In addition, prolapse of the barrier may be mitigated by creating membrane geometry (such as a pleats or slits) to create overlaps, spines of less flexible material to keep the barrier at an angle 90 degrees or below from the central axis, or an excess of material at the trailing end (number 29).

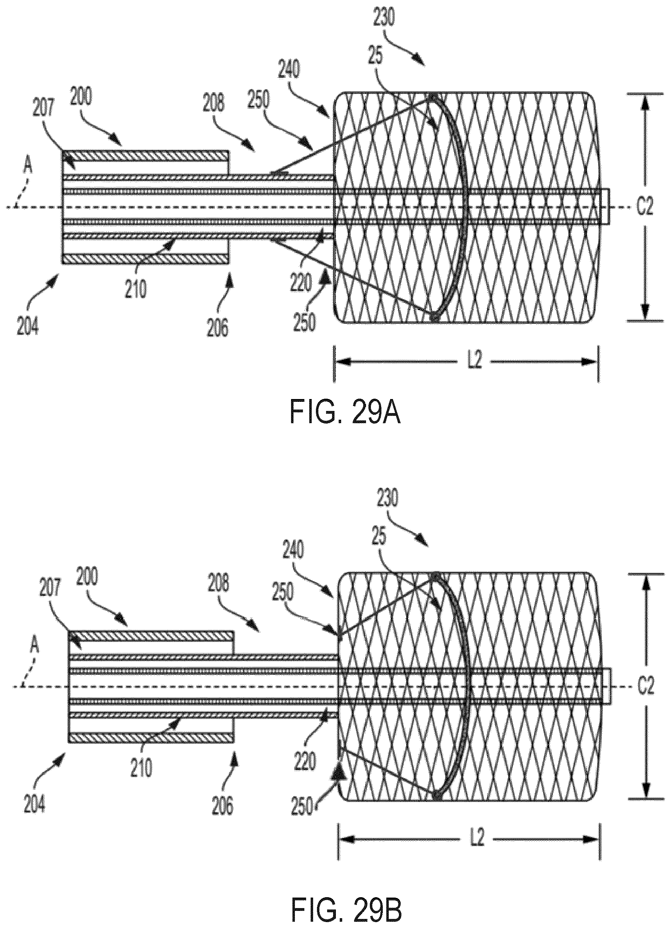

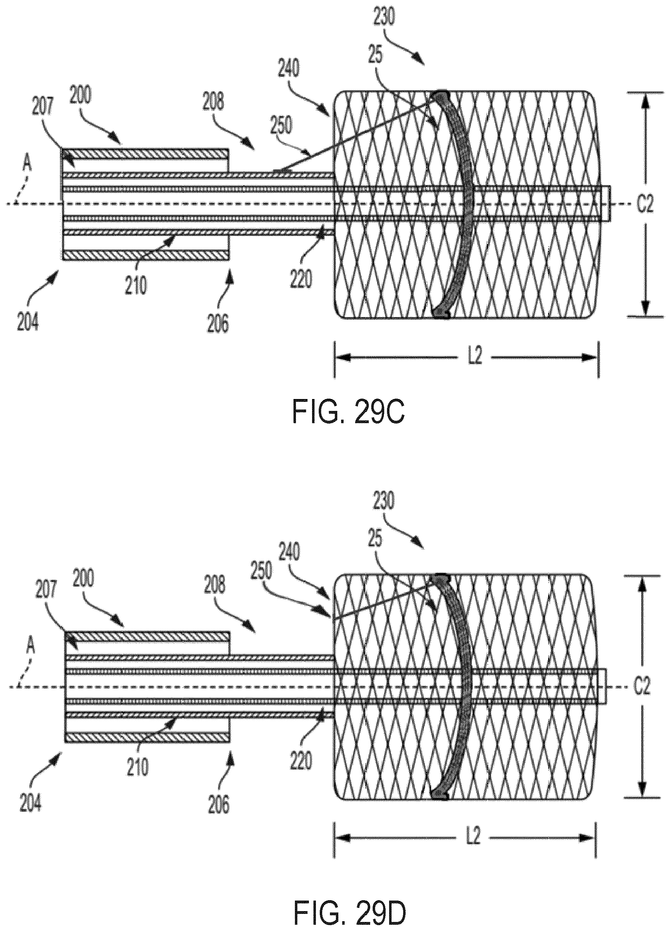

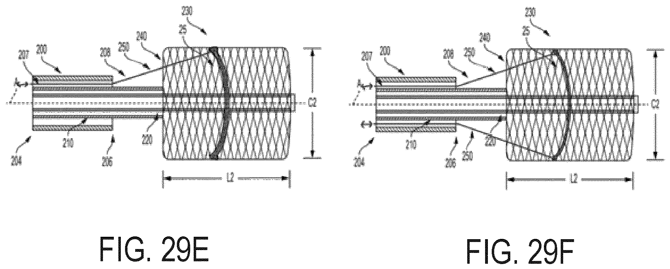

Referring to Figures 29A-29F, in one embodiment, the system 200 includes tethers 250 attached to the barrier 25. The tethers 250 are configured to prevent barrier prolapse during use and prevent barrier protrusion through the valve frame cells during valve assembly collapse and recapture into the outer sheath 204. Referring to Figure 29A, in one embodiment, tethers 250 are connected from the proximal edge of the barrier 25 to the to the distal end of the outer shaft 210. Referring to Figure 29B, in another embodiment, the tethers 250 are connected from the proximal edge of the barrier 25 to more proximal locations of the valve frame 240. Referring to Figure 29C, in another embodiment, a single tether is integrated into the proximal rim of the barrier, in a drawstring like manner, to connect the proximal edge of the barrier to the distal end of the outer shaft. Referring to Figure 29D, alternatively, a single tether is integrated into the proximal rim of the barrier, in a drawstring like manner, to a more proximal location of the valve frame. Referring to Figures 29E and 29F, in another embodiment, the tethers 250 may extend the full length of the catheter system to be actuated by the surgical system handle 202.

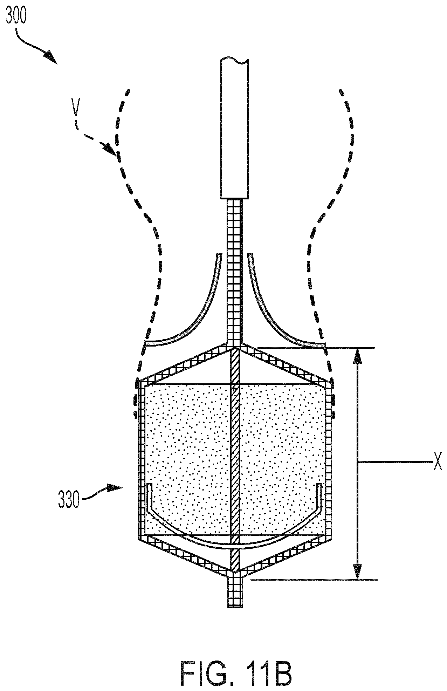

Another embodiment of a surgical system of the present disclosure is illustrated in Figure 11B. As shown, the surgical system 300 is substantially similar to the surgical system illustrated in Figures 9A-11A. Accordingly, common reference numbers are used to identify features that are common to the surgical system 200 and the surgical system 300. As shown in Figure 11B, the surgical system 300 includes an expandable valve assembly 330 that has a length X that extends from the forward end to the trailing end along a central axis. In this case, the length X is sufficient to extend from a bottom of an implanted valve frame into the left ventricular outflow tract (LVOT) when positioned distally to the implanted valve frame, as shown in Figure 11B. In this case, this configuration increases clearance between the barrier 25 and the implanted or native valve leaflets (to minimize risk of interaction between the barrier and the leaflet excision catheter). In addition, this increased length may also increase valve assembly stability/ positioning.

In another embodiment, as shown in Figure 18, the expandable valve assembly 400 may be positioned in LVOT. While the surgical system is shown is a sub-coronary position, the surgical system 300 may be configured to be positioned in the aortic tract, proximal to the coronary ostia, as shown in Figure 23.

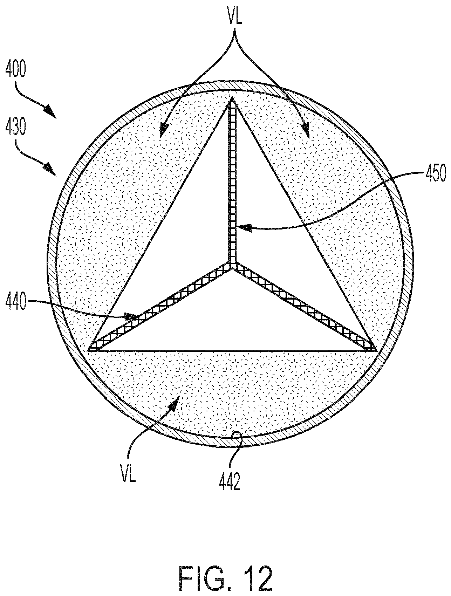

Another embodiment of surgical system 400 is shown in Figure 12. As shown, common reference numbers are used to identify features that are common to the surgical system 200, 300 and the surgical system 400. Figure 12 illustrates the system 400 from an inferior view through the implanted or native valve from the ascending aorta with expandable valve assembly 430 expanded distal or inferior to the valve leaflets VL, which are shown open during systole flow. The surgical system 400 includes an expandable valve assembly 430 having a frame 440. The expandable valve assembly 430 may include at least three frame struts 450 that extend from the outer diameter of the valve assembly 430, which is opposed to the wall of the LVOT 442 when in an expanded configuration, toward a central axis A of the expandable valve assembly 430. The struts 450 may form the forward section and/or trailing section of the valve frame 430. The frame struts 450 are configured and positioned to align with the implanted or native valve commissures to minimize risk of interacting with leaflet excision catheter operation. In certain embodiments, the struts 450 may have a polymeric coating to minimize electrical interference with cutting devices used for leaflet excision that may also have RF cutting functionality. In certain embodiments, the valve assembly may include radiopaque features to aid in proper alignment of the valve assembly relative to the implanted or native valve features.

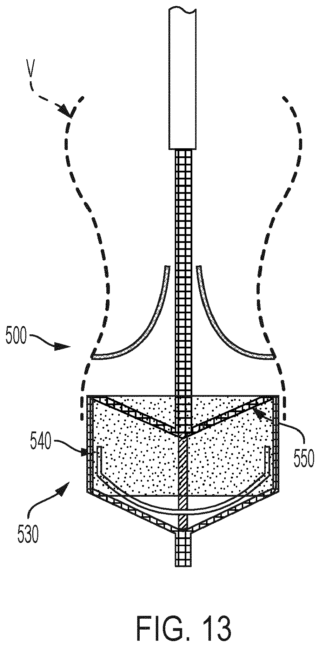

Another embodiment of surgical system 500 is shown in Figure 13. As shown, the surgical system 500 is substantially similar to the surgical system illustrated in Figures 9A-12. Accordingly, common reference numbers are used to identify features that are common to the surgical system 200, 300, 400 and the surgical system 500. The surgical system 500 includes an expandable valve assembly 530 having a frame 540. The valve assembly 530 has an outer wall, a trailing end 550, and a forward end. More specifically, the trailing end 550 extends inwardly from the outer wall and in a distal direction toward the forward end. As such, the trailing end 550 includes a trailing end section that extends inwardly at an angle relative to the outer wall. In this example, the angle is generally less than about 90 degrees. The trailing end 550 is thus prolapsed to minimize interaction or interference with the leaflet excision operation.

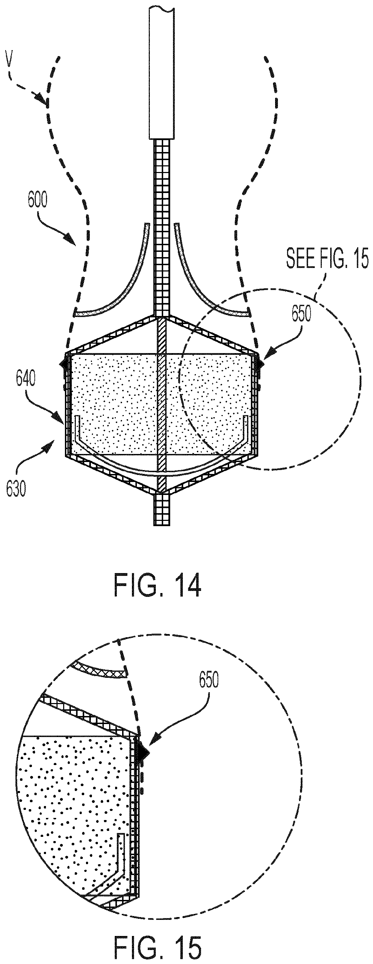

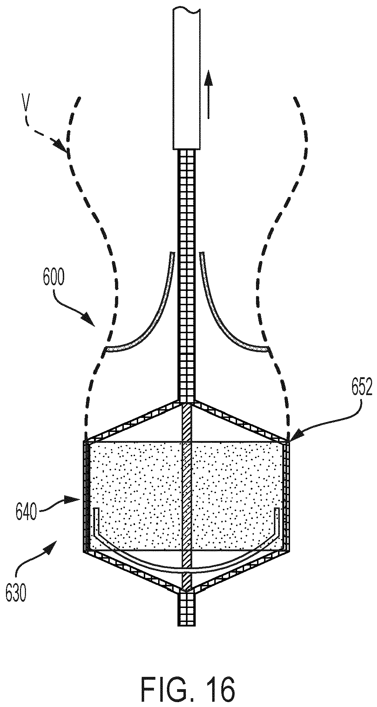

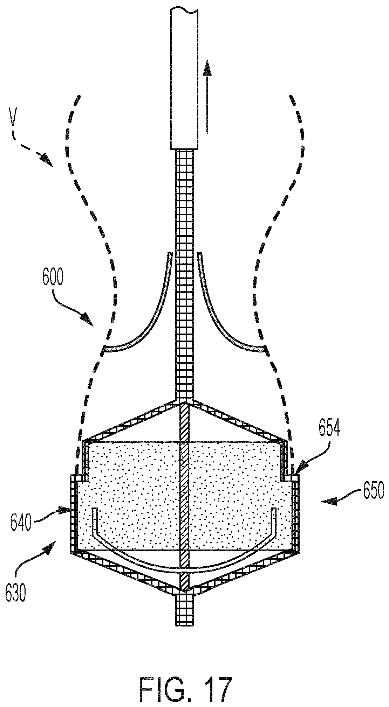

Another embodiment of a surgical system 600 is shown in Figure 14 and Figure 15. As shown, the surgical system 600 is substantially similar to the surgical system illustrated in Figures 9A-13. Accordingly, common reference numbers are used to identify features that are common to the surgical system 200, 300, 400, 500, and the surgical system 600. As shown in Figures 14-17, the surgical system 600 includes an expandable valve assembly 630 having a frame 640 forming an outer wall. The outer wall includes at least one anchor 650 configured to engage with an inner surface of a valve frame. In one example, at least one anchor 650 is a projection that extends outwardly in a direction perpendicular to a central axis of the expandable valve assembly 630. In another example as shown in Figure 16, the trailing end of the frame defines an anchor 652 and is configured to abut a distal terminal edge of a valve frame. In yet another example as shown in Figure 17, the anchor is configured as a ridge 654 that extends at least partially around the outer wall. In this example, the ridge 654 is configured to abut a distal terminal edge of a valve frame. The ridge 654 is spaced apart a distance from the plane that is perpendicular to the outer wall and intersects a forward end of the expandable valve assembly 630. The distance between the ridge 654 and the plane may be selected as needed. The ridge 654, or anchor, may be located along any particular part of the outer wall of the frame 640. With anchors 650, 652, 654, a tension applied to the outer shaft and the inner shaft causes the anchors 650, 652, 654 to engage with or apply an upward force against the distal terminal edge of the valve frame. This, in turn, maintains a position of the expandable valve assembly 630 relative to leaflets of the valve frame.

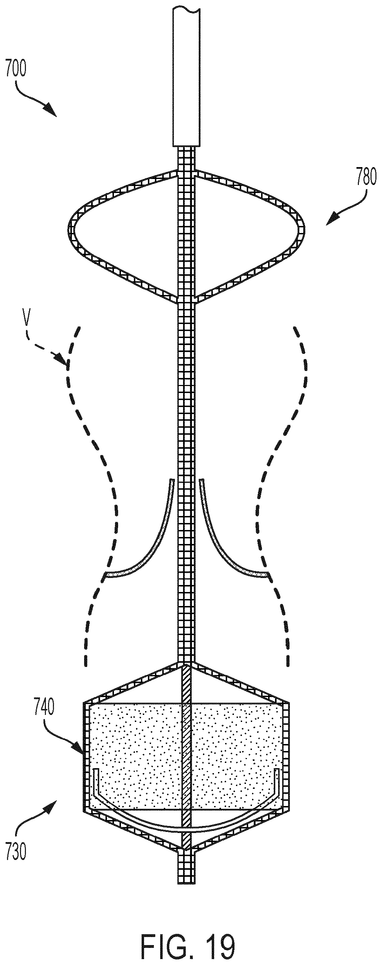

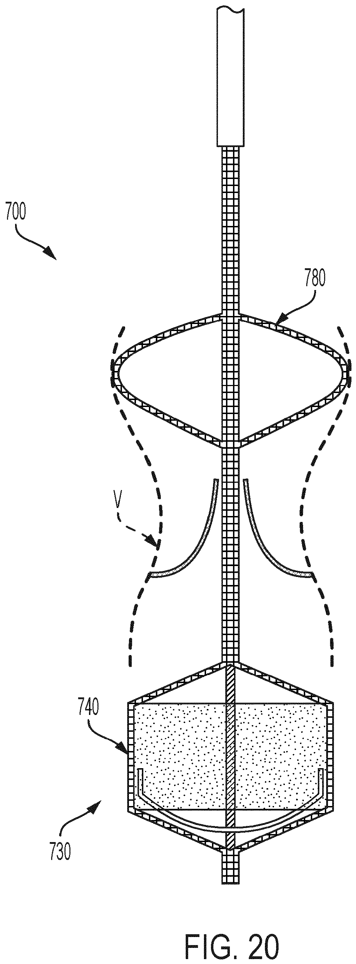

Another embodiment of a surgical system 700 is shown in Figure 19 and Figure 20. As shown, the surgical system 700 is substantially similar to the surgical system illustrated in Figures 9A-18. Accordingly, common reference numbers are used to identify features that are common to the surgical system 200, 300, 400, 500, 600, and the surgical system 700. As shown in Figures 19 and 20, the surgical system 700 includes an expandable valve assembly 730 having a frame 740 forming an outer wall. Also included is one or more, e.g. two to three, support struts 780 configured to expand outwardly away from a central axis A of the outer shaft or the inner shaft. The support struts are there to provide added positioning and positional stability. In addition, they may also serve to hold the target leaflets in the position to ease the excision process. In one example, the support strut is three support struts. Two struts may be used or more than three struts as needed. The support strut may be formed from a shape memory material that expands outward when exiting the outer sheath. The support struts may be located along the inner shaft or the outer shaft as needed.

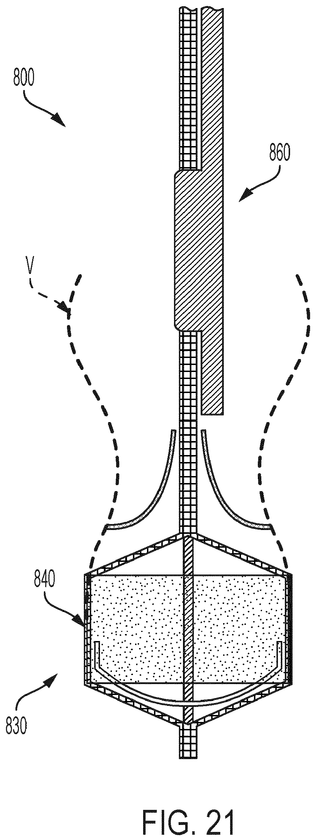

Another embodiment of a surgical system 800 is shown in Figure 21. As shown, the surgical system 800 is substantially similar to the surgical system illustrated in Figures 9A-20. Accordingly, common reference numbers are used to identify features that are common to the surgical system 200, 300, 400, 500, 600, 700, and the surgical system 800. As shown in Figure 21, the surgical system 800 includes an inner shaft, and outer shaft, and an expandable valve assembly 830 having a frame 840. The surgical system 800 further comprising a guide element 860 coupled to the outer shaft. The guide element 860 configured to slidingly guide a separate medical device, such as a TAVR delivery system or a cutting device used for leaflet excision. The element may include an elongated lumen through the separate medical device may pass through. In this example, the guide element may be a monorail design. The lumen may be closed or open. For example, the guide element includes an open groove along which the separate medical device may pass, which may also be referred to as a slitted monorail. The separate medical device is an excision catheter having one or more cutting elements.

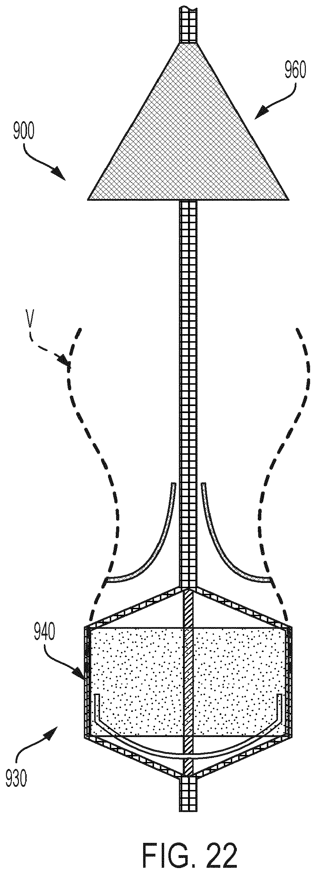

Another embodiment of a surgical system 900 is shown in Figure 22. As shown, the surgical system 900 is substantially similar to the surgical system illustrated in Figures 9A-21. Accordingly, common reference numbers are used to identify features that are common to the surgical system 200, 300, 400, 500, 600, 700, 800, and the surgical system 900. As shown in Figure 22, the surgical system 900 includes an inner shaft, and outer shaft, and an expandable valve assembly 930 having a frame 940. The surgical system further comprising a filter assembly 960 coupled to the outer shaft and configured to expand when the outer sheath is retracted in a proximal direction, exposing the filter assembly, and collapse when the outer sheath is advanced in a distal direction that is opposite the proximal direction. The filter 960 is configured to capture debris as needed. The filter may be used to extract and capture emboli, such as water vapor, char, smoke, oxygen, nitrogen, carbon dioxide, solids, tissue fragments, etc. In one example, the filter may be positioned to oppose the aortic wall in a manner that captures particles from the forward flow ejection of the left ventricle (LV). The filter may also serve to the enhance position and positional stability of the valve assembly.

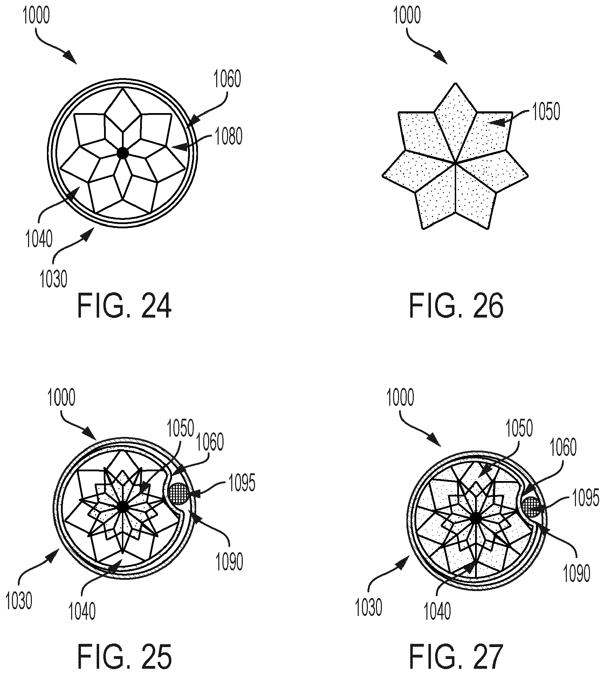

Figures 24-27 illustrates another embodiment of a surgical system that is configured to permit an adjacent catheter to pass along side it while still maintaining adequate control of blood flow through the valve frame. Certain features of the surgical systems shown in Figures 9A-23 may be included in the surgical system shown in Figures 24-27. Accordingly, common reference numbers are used to identify features that are common to the surgical system 200, 300, 400, 500, 600, 700, 800, 900 and the surgical system 1000. As shown, the surgical system 1000 includes an expandable valve assembly 1030 and a flexible skirt 1060 that at least partially surrounds the outer wall of the frame 1040. The surgical system also includes at least one void 1080 between the outer wall of the frame and the flexible skirt 1060. The surgical system also includes a barrier 1050 within the expandable valve assembly that is configured to selectively transition between an open configuration (Figure 25), where the barrier 1050 is collapsed inwardly, and a closed configuration (Figure 27), where the barrier 1050 is expanded outwardly adjacent to the outer wall. The flexible skirt 1060 is configured to conform to the outer wall adjacent the at least one void 1080 to create a passage 1090 external to the flexible skirt 1060 that permits an adjacent catheter 1095 to enter alongside expandable valve assembly 1030. In the embodiment shown, the barrier 1050 may include folds or pleats so that it could more easily conform to the adjacent catheter positioned in the valve frame void. In other embodiment, the barrier would not have such folds or pleats but could be cone or dome shaped as needed.

Another embodiment of a surgical system includes an outer shaft, an inner shaft, and an expandable valve assembly. The outer shaft includes a lumen. The inner shaft is carried in the lumen such that one or both the outer shaft and the inner shaft are movable relative to each other. The expandable valve assembly is deployed within an aortic valve. The aortic valve may be an implanted aortic valve or a native aortic valve. The expandable valve assembly includes a barrier within the expandable valve assembly. The expandable valve assembly is configured to push one or more valve leaflets towards the aorta wall as a diagnostic tool to determine risk of obstruction of blood flow to the coronary arteries upon deployment of a replacement transcatheter heart valve.

Another embodiment of a surgical system includes an outer shaft, an inner shaft, and an expandable valve assembly configured to determine the risk of left ventricular outflow tract obstruction. The outer shaft includes a lumen. The inner shaft is carried in the lumen such that one or both the outer shaft and the inner shaft are movable relative to each other. The expandable valve assembly is deployed within a mitral valve. The mitral valve may be an implanted mitral valve or a native mitral valve. The expandable valve assembly includes a barrier within the expandable valve assembly.

Another embodiment of a surgical system includes an outer shaft, an inner shaft, and an expandable valve assembly configured to dilate a heart valve. The outer shaft includes a lumen. The inner shaft is carried in the lumen such that one or both the outer shaft and the inner shaft are movable relative to each other. The expandable valve assembly is deployed within a heart valve. The heart valve may be an implanted heart valve or a native heart valve. The expandable valve assembly includes a barrier within the expandable valve assembly.

Another embodiment of a surgical system includes an outer shaft, an inner shaft, and an expandable valve assembly configured to improve leaflet mobility. The outer shaft includes a lumen. The inner shaft is carried in the lumen such that one or both the outer shaft and the inner shaft are movable relative to each other. The expandable valve assembly is deployed within a heart valve. The heart valve may be an implanted heart valve or a native heart valve. The expandable valve assembly includes a barrier within the expandable valve assembly. The expandable valve assembly is further configured to create one or more fractures in one or more calcified lesions located on the heart valve. The expandable valve assembly is further configured to separate one or more calcified leaflet commissures located on the heart valve.

Another embodiment of a surgical system for measuring anatomical dimensions of a heart valve. The surgical system includes an outer shaft, an inner shaft, and an expandable valve assembly. The outer shaft includes a lumen. The inner shaft is carried in the lumen such that one or both the outer shaft and the inner shaft are movable relative to each other. The expandable valve assembly is deployed within a heart valve. The heart valve may be an implanted heart valve or a native heart valve. The expandable valve assembly includes a barrier. The expandable valve assembly further includes a proximal handle that includes a scale, wherein the scale is configured to measure and indicate valve frame diameter. In one embodiment, the valve frame may also be utilized as a dimensional reference in conjunction with standard medical imaging modalities (e.g. fluoroscopy, ultrasound or CT) to measure anatomical dimensions of the heart valve.

In any of the embodiments described above, a diameter of the expandable valve assembly is selectively actuated via a proximal handle of the expandable valve assembly. In addition, in any of the embodiments described above, a radial outward force exerted by the expandable valve assembly is selectively actuated via the proximal handle of the expandable valve assembly.

The surgical systems 200-1000 and of any of the additional embodiments as described herein may be used in method to control blood flow in an implanted or native valve during use. More specifically, the method may include placing a surgical system including the outer sheath and the expandable valve assembly in a radial artery. In another variation, the method includes placing a surgical system including the outer sheath and the expandable valve assembly in a femoral artery.

Then, the user advances a distal end of an outer sheath to a location in a heart proximate to an implanted or native valve. In one example, the method would include advancing a distal end of an outer sheath to a location in an ascending aorta proximal to an implanted or native valve. However, the outer sheath may advance to any particular location to position the expandable valve assembly as intended.

The method may include advancing the expandable valve assembly in a collapsed configuration into a heart to a location proximate to an implanted or native valve. In this example, the expandable valve assembly is coupled to an outer shaft and an inner shaft as described above. The user may then position the expandable valve assembly in a sub-coronary location proximate the implanted or native valve. In an alternative method, the user may position the expandable valve assembly in an ascending aorta proximate to the implanted or native valve. In yet another example, the user could position the expandable valve assembly distal to the coronary arteries so that a leading end of the expandable valve assembly is located in a left ventricular outflow tract. The method may include retracting the outer sheath to expose the valve assembly and, as needed later in the procedure, advancing the outer sheath to recapture valve assembly.

A user may actuate movement of one or both of the outer shaft and the inner shaft to transition the expandable valve assembly from the collapsed configuration into an expanded configuration. There are a number of different mechanisms possible to expand the valve assembly. More specifically, advancing the outer shaft relative to the inner shaft to transition the expandable valve assembly into the expanded configuration. Or, retracting the inner shaft relative to the outer shaft to transition the expandable valve assembly into the expanded configuration. In addition, the user could advance the inner shaft relative to the outer shaft to transition the expandable valve assembly into the expanded configuration. Or, the user could retract the outer shaft relative to the inner shaft to transition the expandable valve assembly into the expanded configuration.

The method may include controlling blood flow through the expandable valve assembly via blood flow responsive opening and closing of a barrier contained within the expandable valve assembly when in the expanded configuration. Controlling blood flow through the expandable valve assembly may include via fluid responsive opening and closing of a barrier contained within the expandable valve assembly.

With the expandable valve assembly in the expanded configuration, the method may include excising a portion of a leaflet of the implanted or native valve. Then, the method may include deploying a replacement valve inside the implanted or native valve.

At the conclusion of the excision or other surgical procedure, the method may include collapsing the expandable valve assembly into collapsed configuration. More specifically, the method may include actuating movement of one or both of the outer shaft and the inner shaft to transition the expandable valve assembly from the expanded configuration into the collapsed configuration. In this case, the user may retract the expandable valve assembly in the collapsed configuration from the location proximate to the implanted or native valve.

In certain embodiments, prior to controlling blood flow, the method includes engaging an implanted valve with an engagement element along an outer wall of the expandable valve assembly to maintain position of the expandable valve relative to the implanted valve. In a variation of this embodiment, the method may include placing a ridge along an outer wall of the expandable valve assembly adjacent to a distal end of the implanted valve and applying tension to the outer shaft or the inner shaft to maintain position of the expandable valve assembly.

Furthermore, prior to the expanded valve assembly, the method may include causing at least one support strut to expand outwardly away from a central axis of the outer shaft or the inner shaft into contact with an inner surface of the implanted valve or inner surface of the ascending aorta superior to an implanted or native valve. This may help center the expandable assembly in the implanted valve.

In yet another embodiment, the method may include inserting a medical device into a guide element located along the outer shaft or the inner shaft when the expandable valve assembly is in the expanded configuration.

Another embodiment of the present disclosure includes advancing an expandable valve assembly in a collapsed configuration to a location in an ascending aorta that is proximal to an implanted or native valve. Then, the user may actuate the expandable valve assembly to transition from the collapsed configuration into an expanded configuration. In this example, the expandable valve assembly has an outer wall, a flexible skirt, and a void formed between the outer wall and the flexible skirt. The user may then insert a separate catheter into a passage formed between the ascending aorta and an outer surface of the flexible skirt. Here, the passage is located where the flexible skirt conforms to the outer wall at the void of the expandable valve assembly to define the passage. The user may perform a surgical procedure with the separate catheter. Next the user may collapse the expandable valve assembly into the collapsed configuration and retract the expandable valve assembly in the collapsed configuration from the ascending aorta.

Another embodiment may include additional steps. Such as the following exemplary steps that may be used in alone or in combination with other method step disclosed herein. The method may include advancing a distal end of an outer sheath to a location in a heart proximate a heart valve, wherein the outer sheath carries the expandable valve assembly.

The method may include advancing a distal end of an outer sheath to a location in an ascending aorta proximal to a heart valve, wherein the outer sheath carries the expandable valve assembly.

The method may include actuating movement of one or both of an outer shaft and an inner shaft, coupled to the expandable valve assembly, to transition the expandable valve assembly from the expanded configuration into the collapsed configuration.

The method may include controlling blood flow through the expandable valve assembly via responsive opening and closing of a barrier contained within the expandable valve assembly.

The method may include actuating movement of one or both the outer shaft and the inner shaft to transition the expandable valve assembly from the expanded configuration into the collapsed configuration.

The method may include, with the expandable valve assembly in the expanded configuration, excising a portion of a leaflet of the heart valve.

The method may include deploying a replacement heart valve inside the heart valve. The method may include causing an expandable valve assembly carried by an outer sheath to exit a distal end of the outer sheath. ere, causing the expandable valve assembly carried by the outer sheath to exit the distal end of the outer sheath includes positioning the expandable valve assembly in a sub-coronary location proximate the heart valve. The method may also include causing the expandable valve assembly carried in a lumen of the outer sheath to exit the distal end of the outer sheath includes positioning the expandable valve assembly in an ascending aorta proximate the heart valve.

The method may include placing a surgical system including the outer sheath and the expandable valve assembly in a radial artery.

The method may include placing a surgical system including the outer sheath and the expandable valve assembly in a femoral artery.

The method step of actuating movement of one or both the outer shaft and the inner shaft may include advancing the outer shaft relative to the inner shaft to transition the expandable valve assembly into the expanded configuration.

The method step of actuating movement of one or both the outer shaft and the inner shaft may include retracting the inner shaft relative to the outer shaft to transition the expandable valve assembly into the expanded configuration.

The method step of actuating movement of one or both the outer shaft and the inner shaft may include advancing the inner shaft relative to the outer shaft to transition the expandable valve assembly into the collapsed configuration.

The method step of actuating movement of one or both the outer shaft and the inner shaft may include retracting the outer shaft relative to the inner shaft to transition the expandable valve assembly into the collapsed configuration.

In the method, the expandable valve assembly has a length that extends from a forward end to a trailing end along a central axis. The method may include positioning the expandable valve assembly distally to the coronary arteries so that a leading end of the expandable valve assembly is located in a left ventricular outflow tract.

The method may include engaging an inner heart valve with an engagement element along an outer wall of the expandable valve assembly to maintain position of the expandable valve relative to the heart valve.

The method may include comprising placing a ridge along an outer wall of the expandable valve assembly adjacent to a distal end of the heart valve and applying tension to the outer shaft or the inner shaft to maintain position of the expandable valve assembly.

The method may include placing the trailing end of the expandable valve assembly adjacent to a distal end of the heart valve and applying tension to the outer shaft or the inner shaft to maintain position of the expandable valve assembly.

The method may include causing at least one support strut to expand outwardly away from a central axis of the outer shaft or the inner shaft into contact with an inner surface of the heart valve or ascending aorta.

The method may include inserting a medical device into a guide element located along the outer shaft or the inner shaft when the expandable valve assembly is in the expanded configuration.

In the method, the expandable valve assembly includes a frame with frame members defining cellular voids.

The method may include aligning frame struts with the valve commissures of the heart valve leaflets.

The method may include prolapsing the trailing end of the valve frame toward the forward section of the valve frame.

The method may include operating a filter coupled to the outer shaft or inner shaft to capture any debris.

Another embodiment of the present disclosure includes a method. The method includes deploying a surgical system within the aortic valve. The method further includes positioning an expandable valve assembly of the surgical system with the aortic valve. The method further includes expanding the expandable valve assembly to push the one or more valve leaflets towards the aorta wall. The method further includes injecting contrast to assess blood flow to coronary arteries. In one embodiment, the contrast is injected via the inner shaft or the surgical system. In an alternative embodiment, the contrast is injected via an ancillary catheter.

Another embodiment of the present disclosure is a method. The method includes deploying the surgical system within the mitral valve. The method further includes positioning an expandable valve assembly of the surgical system with the mitral valve. The method further includes expanding the expandable valve assembly to push the one or more valve leaflets outwardly to determine the risk of left ventricular outflow tract obstruction.

It will be appreciated by those skilled in the art that various modifications and alterations of the present disclosure may be made without departing from the broad scope of the appended claims. Some of these have been discussed above and others will be apparent to those skilled in the art. The scope of the present disclosure is limited only by the claims. A method, comprising deploying the surgical system according to the present disclosure within the mitral valve; positioning the expandable valve assembly of the surgical system with the mitral valve, expanding the expandable valve assembly to push the one or more valve leaflets outwardly to determine the risk of left ventricular outflow tract obstruction.

### The present invention is defined at least in part by the following numbered paragraphs

1. A surgical system, comprising:
   an outer shaft having a lumen;
   an inner shaft carried in the lumen such that one or both the outer shaft and the inner shaft are movable relative to each other; and
   an expandable valve assembly including a barrier within the expandable valve assembly and configured to selectively permit or inhibit fluid flow through the expandable valve assembly when in an expanded configuration, the expandable valve assembly being coupled to the outer shaft and the inner shaft, such that, movement of one or both the outer shaft and the inner shaft cause the expandable valve assembly to transition between a collapsed configuration and the expanded configuration.
2. A surgical system, comprising:
   an outer shaft having a lumen;
   an inner shaft carried in the lumen such that one or both the outer shaft and the inner shaft are movable relative to each other; and
   an expandable valve assembly having a forward end and a trailing end, the forward end being coupled to the inner shaft and the trailing end being coupled to the outer shaft, such that, movement of either or both the inner shaft and the outer shaft cause the expandable valve assembly to transition between a collapsed configuration and an expanded configuration.
3. A surgical system, comprising:
   an outer shaft having a lumen;
   an inner shaft carried in the lumen such that one or both the outer shaft and the inner shaft are movable relative to each other; and
   an expandable valve assembly coupled to one or both of the outer shaft and the inner shaft, such that, movement of one or both the outer shaft and the inner shaft is configured to 1) cause the expandable valve assembly to transition between a collapsed configuration and an expanded configuration, and 2) selectively control an outer cross-sectional dimension of the expandable valve assembly, thereby selectively adjusting the radial forces applied outwardly by the expandable valve assembly.
4. A surgical system, comprising:
   an expandable valve assembly having a forward end, a trailing end, an outer wall, a flexible skirt that at least partially surrounds the outer wall, and at least one void between the outer wall and the flexible skirt; and
   a barrier within the expandable valve assembly and configured to selectively transition between an open configuration, where the barrier is collapsed inwardly, and a closed configuration, where the barrier is expanded outwardly adjacent to the outer wall, wherein the flexible skirt is configured to conform to the outer wall adjacent the at least one void to create passage external to the flexible skirt that permits an adjacent catheter to enter alongside an expandable valve assembly.
5. The surgical system according to paragraph 4, further comprising:
   an outer shaft having a lumen; and
   an inner shaft carried in the lumen such that one or both the outer shaft and the inner shaft are movable relative to each other.
6. The surgical system according to any one of paragraphs 1 to 5, further comprising an outer sheath having a distal end and a channel that extends therethrough, wherein the expandable valve assembly is moveable from within the channel to a location outside of the channel.
7. The surgical system according to any one of paragraphs 1 to 6, wherein the forward end is coupled to the inner shaft and the trailing end is coupled to the outer shaft, such that, movement of either or both the inner shaft and the outer shaft cause the expandable valve assembly to transition between the collapsed configuration and the expanded configuration.
8. The surgical system according to any one of paragraphs 1 to 6, wherein the outer shaft has a proximal end and a distal end, and the inner shaft has a proximal end and a distal end, wherein the forward end of the expandable valve assembly is coupled to the distal end of the inner shaft and the trailing end of the expandable valve assembly is coupled to the distal end of the outer shaft, such that, movement of either or both the inner shaft and the outer shaft causes the expandable valve assembly transition between the collapsed configuration and the expanded configuration.
9. The surgical system according to any one of paragraphs 1 to 8 and paragraph 15, wherein the expandable valve assembly in the collapsed configuration has a first outer cross-sectional dimension and expandable valve assembly in the expanded configuration has a second outer cross-sectional dimension that is greater than the first outer cross-sectional dimension.
10. The surgical system according to any one of paragraphs 1 to 9 and paragraph 15, wherein the barrier is configured to transition between an open configuration, where fluid flow is capable of passing through the expandable valve assembly, and a closed configuration, where fluid flow is substantially inhibited from passing through the expandable valve assembly, when the expandable valve assembly is in the expanded configuration.
11. The surgical system according to any one of the preceding paragraphs, wherein advancement of the outer shaft relative to the inner shaft causes the expandable valve assembly to transition from the collapsed configuration to the expanded configuration.
12. The surgical system according to any one of the preceding paragraphs, wherein retraction of the inner shaft relative to the outer shaft causes the expandable valve assembly to transition from the collapsed configuration to the expanded configuration.
13. The surgical system according to any one of the preceding paragraphs, wherein advancement of the inner shaft relative to the outer shaft causes the expandable valve assembly to transition from the expanded configuration into the collapsed configuration.
14. The surgical system according to any one of the preceding paragraphs, wherein retraction of the outer shaft relative to the inner shaft causes the expandable valve assembly to transition from the expanded configuration to the collapsed configuration.
15. A surgical system, comprising:
   an outer sheath having a lumen;
   an outer shaft carried in the lumen such that one or both the outer sheath and the outer shaft are movable relative to each other; and;
   an expandable valve assembly including forward end, a trailing end, and a barrier within the expandable valve assembly and configured to selectively permit or inhibit fluid flow through the expandable valve assembly when in an expanded configuration, the forward end of the expandable valve assembly being coupled to the outer shaft and the trailing end being uncoupled to the outer shaft, such that, movement of the outer sheath relative to the outer shaft causes the expandable valve assembly to transition between a collapsed configuration and an expanded configuration.
16. The surgical system according to any one of the preceding paragraphs, wherein the expandable valve assembly has a length that extends from the forward end to the trailing end along a central axis, wherein the length is sufficient to extend from a bottom of an implanted valve frame into the left ventricular outflow tract when positioned distally to the implanted valve frame.
17. The surgical system according to any one of the preceding paragraphs, further comprising at least three frame struts that extend from an outer wall of the expandable valve assembly toward a central axis of the expandable valve assembly.
18. The surgical system according to paragraph 17, wherein the expandable valve assembly includes a forward section comprising the at least three frame struts.
19. The surgical system according to paragraph 18, wherein the expandable valve assembly includes a trailing section comprising at least three frame struts.
20. The surgical system according to any one of the preceding paragraphs, further comprising a polymeric coating on the expandable valve assembly.
21. The surgical system according to any one of the preceding paragraphs, wherein the expandable valve assembly has an outer wall, and a trailing end of the expandable valve assembly that extends inwardly from the outer wall and in a distal direction toward the leading end.
22. The surgical system according to any one of the preceding paragraphs, wherein the expandable valve assembly has an outer wall and at least one anchor configured to engage with an inner surface of a valve frame.
23. The surgical system according to any one of the preceding paragraphs, wherein the at least one anchor is a projection that extends outwardly in a direction perpendicular to a central axis of the expandable valve assembly.
24. The surgical system according to any one of the preceding paragraphs, wherein the at least one anchor is a ridge that extends at least partially around the outer wall, wherein the ridge is configured to abut a distal terminal edge of a valve frame.
25. The surgical system according to paragraph 24, wherein tension applied to the outer shaft and the inner shaft and the ridge against the distal terminal edge of the valve frame maintains a position of the expandable valve assembly relative to leaflets of the valve frame, where the ridge is spaced apart a distance from the plane that is perpendicular to the outer wall and intersects a forward end of the expandable valve assembly.
26. The surgical system according to any one of paragraphs 22 to 24, wherein tension applied to the outer shaft and the inner shaft and expandable valve assembly against the distal terminal edge of the valve frame maintains a position of the expandable valve assembly relative to leaflets of the valve frame.
27. The surgical system according to any one of paragraphs 1, 2, and 4 to 21, further comprising a flexible skirt affixed to the outer wall of the expandable valve assembly, wherein the flexible skirt is configured to provide sealing engagement with the barrier, an inner surface of an implanted valve frame, the inner surface of an LVOT, or the inner surface of the ascending aorta.
28. The surgical system according to any one of the preceding paragraphs, further comprising a support strut configured to expand outwardly away from a central axis of the outer shaft or the inner shaft.
29. The surgical system according to paragraph 28, wherein the support strut is two or more support struts.
30. The surgical system according to paragraph 28 or paragraph 29, wherein the support strut is formed from a shape memory material.
31. The surgical system according to any one of the preceding paragraphs, further comprising a guide element coupled to the outer shaft, wherein the guide element is configured to slidingly guide a separate medical device.
32. The surgical system according to paragraph 31, wherein the guide element includes an elongated lumen that the separate medical device passes through.
33. The surgical system according to paragraph 31, wherein the guide element includes an open slit along which the separate medical device passes.
34. The surgical system according to paragraph 31, wherein the separate medical device is an excision catheter having one or more cutting elements.
35. The surgical system according to any one of the preceding paragraphs, further comprising a filter coupled to the outer shaft and configured to expand when the outer sheath is retracted in a proximal direction, and collapse when the outer sheath is advanced in a distal direction that is opposite the proximal direction.
36. The surgical system according to any one of the preceding paragraphs, wherein the expandable valve assembly includes an expandable frame formed from a shape memory material.
37. The surgical system according to any one of the preceding paragraphs, wherein the expandable valve assembly includes a frame formed from a laser cut metallic alloy.
38. The surgical system according to any one of the preceding paragraphs, wherein the expandable valve assembly includes an outer wall, and the forward end defines a forward section that extends from the outer wall toward a forward tip that is coupled to inner shaft, and the trailing end defines a trailing section that extends from the outer wall toward a trailing tip that is coupled to the outer shaft, wherein an optional skirt is affixed to the outer wall but does not overlay the forward section or the trailing section.
39. The surgical system according to any one of the preceding paragraphs, wherein the outer wall, forward section, and trailing section are formed by multiple frame members that define cellular voids.
40. The surgical system according to any one of the preceding paragraphs, wherein the valve frame is positioned such that native or bioprosthetic valve leaflets are moved into an orientation that enables or eases the laceration or excision of a valve leaflet.
41. The surgical system according to any one of the preceding paragraphs, further comprising one or more tethers attached to the barrier, the one or more tethers configured to a) prevent barrier prolapse during use and b) prevent barrier protrusion through the valve frame during valve assembly collapse.
42. The surgical system according to paragraph 41, wherein the tethers are connected from a proximal edge of the barrier to the to the distal end of the outer shaft.
43. The surgical system according to paragraph 41, wherein the tethers are connected from a proximal edge of the barrier to a more proximal portion of the valve frame.
44. The surgical system according to paragraph 41, wherein a single tether is integrated into a proximal rim of the barrier, such that the proximal rim of the barrier is connected to the distal end of the outer shaft.
45. The surgical system according to paragraph 41, wherein a single tether is integrated into a proximal rim of the barrier to a proximal portion of the valve frame.
46. The surgical system of paragraph 41, wherein the tethers extend along the length of the catheter system and are actuated by a handle.
47. A surgical system, comprising:
   an outer shaft having a lumen;
   an inner shaft carried in the lumen such that one or both the outer shaft and the inner shaft are movable relative to each other; and
   an expandable valve assembly deployed within an aortic valve and including a barrier within the expandable valve assembly, wherein the expandable valve assembly is configured to push one or more valve leaflets towards the aorta wall to determine risk of obstruction of blood flow to the coronary arteries.
48. A surgical system, comprising:
   an outer shaft having a lumen;
   an inner shaft carried in the lumen such that one or both the outer shaft and the inner shaft are movable relative to each other; and
   an expandable valve assembly deployed within a mitral valve and including a barrier within the expandable valve assembly, wherein the expandable valve assembly is configured to determine the risk of left ventricular outflow tract obstruction.
49. A surgical system, comprising:
   an outer shaft having a lumen;
   an inner shaft carried in the lumen such that one or both the outer shaft and the inner shaft are movable relative to each other; and
   an expandable valve assembly deployed within a heart valve and including a barrier within the expandable valve assembly, wherein the expandable valve assembly is configured to dilate the heart valve.
50. A surgical system, comprising:
   an outer shaft having a lumen;
   an inner shaft carried in the lumen such that one or both the outer shaft and the inner shaft are movable relative to each other; and
   an expandable valve assembly deployed within a heart valve and including a barrier within the expandable valve assembly, wherein the expandable valve assembly is configured to improve leaflet mobility.
51. The surgical system according to paragraph 50, wherein the expandable valve assembly is further configured to create one or more fractures in one or more calcified lesions located on the heart valve.
52. The surgical system according to paragraph 50, wherein the expandable valve assembly is further configured to separate one or more calcified leaflet commissures located on the heart valve.
53. A surgical system for measuring anatomical dimensions of a heart valve, comprising:
   an outer shaft having a lumen;
   an inner shaft carried in the lumen such that one or both the outer shaft and the inner shaft are movable relative to each other; and
   an expandable valve assembly deployed within the heart valve and including a barrier, the expandable valve assembly having a proximal handle that includes a scale, wherein the scale is configured to measure and indicate valve frame diameter.
54. The surgical system according to paragraph 53, wherein the valve frame is utilized as a dimensional reference in conjunction with standard medical imaging modalities to measure anatomical dimensions of the heart valve.
55. The surgical system according to any one of paragraphs 1 to 54, wherein the expandable valve assembly includes one or more radiopaque markers configured to align the valve assembly with features of an existing implanted valve or surrounding native anatomy.
56. The surgical system according to any one of paragraphs 1 to 55, wherein a diameter of the expandable valve assembly is selectively actuated via a proximal handle of the expandable valve assembly.
57. The surgical system according to any one of paragraphs 1 to 56, wherein a radial outward force exerted by the expandable valve assembly is selectively actuated via a proximal handle of the expandable valve assembly.

## Claims

1. A surgical system, comprising:
an outer shaft having a lumen;
an inner shaft carried in the lumen such that one or both the outer shaft and the inner shaft are movable relative to each other; and
an expandable valve assembly including a barrier within the expandable valve assembly and configured to selectively permit or inhibit fluid flow through the expandable valve assembly when in an expanded configuration, the expandable valve assembly being coupled to the outer shaft and the inner shaft, such that, movement of one or both the outer shaft and the inner shaft cause the expandable valve assembly to transition between a collapsed configuration and the expanded configuration.

2. The surgical system according to claim 1, wherein:
movement of one or both the outer shaft and the inner shaft is configured to selectively control an outer cross-sectional dimension of the expandable valve assembly, thereby selectively adjusting the radial forces applied outwardly by the expandable valve assembly.

3. The surgical system according to claim 1, further comprising:
a barrier within the expandable valve assembly and configured to selectively transition between an open configuration, where the barrier is collapsed inwardly, and a closed configuration, where the barrier is expanded outwardly adjacent to the outer wall, wherein the flexible skirt is configured to conform to the outer wall adjacent the at least one void to create passage external to the flexible skirt that permits an adjacent catheter to enter alongside an expandable valve assembly.

4. The surgical system according to claim 1 to 3, wherein the forward end is coupled to the inner shaft and the trailing end is coupled to the outer shaft, such that, movement of either or both the inner shaft and the outer shaft cause the expandable valve assembly to transition between the collapsed configuration and the expanded configuration.

5. The surgical system according to any one of claims 1 to 4, wherein the barrier is configured to transition between an open configuration, where fluid flow is capable of passing through the expandable valve assembly, and a closed configuration, where fluid flow is substantially inhibited from passing through the expandable valve assembly, when the expandable valve assembly is in the expanded configuration.

6. The surgical system according to any one of the preceding claims, wherein the expandable valve assembly has a length that extends from the forward end to the trailing end along a central axis, wherein the length is sufficient to extend from a bottom of an implanted valve frame into the left ventricular outflow tract when positioned distally to the implanted valve frame.

7. The surgical system according to any one of the preceding claims, wherein the expandable valve assembly has an outer wall, and a trailing end of the expandable valve assembly that extends inwardly from the outer wall and in a distal direction toward the leading end.

8. The surgical system according to any one of the preceding claims, wherein the expandable valve assembly has an outer wall and at least one anchor configured to engage with an inner surface of a valve frame.

9. The surgical system according to any one of claims 1 to 7, further comprising a flexible skirt affixed to the outer wall of the expandable valve assembly, wherein the flexible skirt is configured to provide sealing engagement with the barrier, an inner surface of an implanted valve frame, the inner surface of an LVOT, or the inner surface of the ascending aorta.

10. The surgical system according to any one of the preceding claims, further comprising a guide element coupled to the outer shaft, wherein the guide element is configured to slidingly guide a separate medical device.

11. The surgical system according to any one of the preceding claims, further comprising a filter coupled to the outer shaft and configured to expand when the outer sheath is retracted in a proximal direction, and collapse when the outer sheath is advanced in a distal direction that is opposite the proximal direction.

12. The surgical system according to any one of the preceding claims, wherein the expandable valve assembly includes an outer wall, and the forward end defines a forward section that extends from the outer wall toward a forward tip that is coupled to inner shaft, and the trailing end defines a trailing section that extends from the outer wall toward a trailing tip that is coupled to the outer shaft, wherein an optional skirt is affixed to the outer wall but does not overlay the forward section or the trailing section.

13. The surgical system according to claim 1,
wherein the expandable valve assembly is configured to be deployed within an aortic valve and includes a barrier within the expandable valve assembly, wherein the expandable valve assembly is configured to push one or more valve leaflets towards the aorta wall to determine risk of obstruction of blood flow to the coronary arteries.

14. The surgical system according to claim 1,
wherein the expandable valve assembly is configured to determine the risk of left ventricular outflow tract obstruction.

15. The surgical system according to any one of claims 1 to 14, wherein a diameter of the expandable valve assembly is selectively actuated via a proximal handle of the expandable valve assembly; and/or
wherein a radial outward force exerted by the expandable valve assembly is selectively actuated via a proximal handle of the expandable valve assembly.
